(19) **Europäisches Patentamt / European Patent Office / Office européen des brevets**

(11) **EP 1 384 444 B1**

(12) **EUROPÄISCHE PATENTSCHRIFT**

(45) Veröffentlichungstag und Bekanntmachung des Hinweises auf die Patenterteilung:
**16.04.2008 Patentblatt 2008/16**

(51) Int Cl.:
*A61B 17/22* (2006.01)　　*A61B 8/08* (2006.01)

(21) Anmeldenummer: **03012811.0**

(22) Anmeldetag: **05.06.2003**

(54) **Lithotripter**

Lithotripter

Lithotripteur

(84) Benannte Vertragsstaaten:
**AT BE BG CH CY CZ DE DK EE ES FI FR GB GR HU IE IT LI LU MC NL PT RO SE SI SK TR**

(30) Priorität: **26.07.2002 DE 10234144**

(43) Veröffentlichungstag der Anmeldung:
**28.01.2004 Patentblatt 2004/05**

(73) Patentinhaber: **Dornier MedTech Systems GmbH 82234 Wessling (DE)**

(72) Erfinder: **Bohris, Christian, Dr. 82152 Krailling (DE)**

(74) Vertreter: **Grünecker, Kinkeldey, Stockmair & Schwanhäusser Anwaltssozietät Maximilianstrasse 58 80538 München (DE)**

(56) Entgegenhaltungen:
US-A- 4 819 621　　US-A- 5 287 856
US-A- 5 658 239

• FOSTER S G ET AL: "FLOW VELOCITY PROFILE VIA TIME-DOMAIN CORRELATION: ERROR ANALYSIS AND COMPUTER SIMULATION" , IEEE TRANSACTIONS ON ULTRASONICS, FERROELECTRICS AND FREQUENCY CONTROL, IEEE INC. NEW.YORK, US, VOL. 37, NR. 3, PAGE(S) 164-175 XP000142196 ISSN: 0885-3010 * abstract *

**EP 1 384 444 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die vorliegende Erfindung betrifft einen Lithotripter zur Fragmentierung eines Zielobjekts, insbesondere eines Konkrements, in einem vorzugsweise menschlichen Körper, umfassend einen Stoßwellengenerator zur Erzeugung fokussierter Stoßwellen, eine Ultraschall-Sende-/Empfangseinheit mit einem Ultraschall-Transducer zum Aussenden von Ultraschallwellen in den Körper und zum Empfangen von in einem Zielgebiet des Stoßwellengenerators reflektierten Ultraschallwellen, und eine an die Ultraschall-Sende-/Empfangseinheit angeschlossene Auswerteeinheit zum Auswerten der empfangenen Ultraschallwellen.

Lithotripter sind heutzutage als medizinische Geräte zur Zertrümmerung von Konkrementen, beispielsweise Nierensteinen, im Körper eines Patienten mit Hilfe fokussierter Stoßwellen weitverbreitet. Derartige Lithotripter werden von der Anmelderin gewerblich angeboten, beispielsweise unter der Bezeichnung "Dornier Lithotripter S" oder "Dornier Compact Delta". Bei all diesen Geräten muß das Konkrement vor Beginn der Behandlung geortet werden, damit der Patient mit Hilfe einer verschiebbaren Liege so positioniert werden kann, daß sich beispielsweise sein Nierenstein im Fokus der Stoßwellen befindet, die mit Hilfe des Stoßwellengenerators des Lithotripters erzeugt werden. Diese anfängliche "Justage", d.h. Positionierung des Patienten erfolgt üblicherweise mit einer bildgebenden Ortungsvorrichtung, etwa einem bildgebenden Ultraschall-Scanner oder einer Röntgenortungsvorrichtung. Diese dient zusätzlich zur anfänglichen erstmaligen Ortung des Konkrements vor Beginn der ESWL-Behandlung (extrakorporale Stoßwellenlithotripsie) oder der ESWT-Behandlung (extrakorporale Stoßwellentherapie) auch dazu, während des Verlaufs der Behandlung die Position des Konkrements kontinuierlich zu überwachen, um sicherzustellen, daß es nicht im Körper des Patienten verrutscht bzw. an einen anderen Ort gewandert ist oder daß sich der Patient nicht auf seiner Liege bewegt hat, so daß sich das Konkrement möglicherweise nicht mehr im Fokus der Stoßwellen befindet. Für einen umfassenden Überblick über technische und medizinische Aspekte der ESWT und der bei Lithotriptem eingesetzten Geräte wird auf das Buch "ESWT and Ultrasound Imaging of the Musculosceletal System", Steinkopff-Verlag, Darmstadt, 2001, ISBN 3-7985-1252-3 verwiesen.

Üblicherweise wird die genannte bildgebende Ortungsvorrichtung auch dazu verwendet, während des Verlaufs der Behandlung den Erfolg der Lithotripsie zu überwachen, d.h. die Fragmentierung des Zielobjekts. Da eine Behandlung typischerweise ca. 30 Minuten dauert, kann wegen der zu hohen Strahlenbelastung nicht kontinuierlich geröntgt werden, sondern allenfalls in Intervallen von 3 bis 5 Minuten. Verlagert sich in der Zwischenzeit das Zielobjekt zum Beispiel durch eine Bewegung des Patienten, so wird bis zur nächsten Kontrollaufnahme der Körper durch Stoßwellen belastet, ohne daß das Zielobjekt weiter fragmentiert wird, da es sich nicht mehr im Stoßwellenfokus befindet.

Bei Lithotriptern, bei denen als bildgebende Ortungsvorrichtung ein Ultraschallscanner eingesetzt wird, kann dieser zwar kontinuierlich zur Visualisierung des Zielobjekts eingesetzt werden, allerdings ist die Ortung u.a.wegen der Bildqualität oft wesentlich schwieriger als bei Röntgenaufnahmen, so daß selbst erfahrenes medizinisches Personal oft Schwierigkeiten hat, das Zielobjekt zu erkennen oder gar seinen Fragmentierungsgrad einzuschätzen.

Darüberhinaus hat die Verwendung derartiger bildgebender Ortungsvorrichtungen nicht nur zur "Anfangsjustage" des Patienten, sondern auch zur "Trefferkontrolle" im Verlauf der Behandlung folgenden grundsätzlichen Nachteil: Zwar läßt sich hierdurch die Position des Zielobjekts relativ zum Stoßwellenfokus kontrollieren, d.h. eine rein geometrische Größe; es läßt sich jedoch nicht die Wirkung der Stoßwellen auf das Zielobjekt selbst feststellen. Grundsätzliche Probleme wie eine mangelnde Ankopplung des Stoßwellengeräts an den Körper des Patienten, eine Abschattung von Stoßwellen, beispielsweise durch Rippen etc., werden daher unter Umständen erst spät erkannt, wenn nämlich im Therapieverlauf keinerlei Effekte am Zielobjekt sichtbar werden.

Daher wurden im Stand der Technik bereits spezielle Ultraschall-Verfahren, insbesondere Ultraschall-Dopplerverfahren zur kontinuierlichen Trefferkontrolle vorgeschlagen. Es wurde nämlich angenommen, daß das Konkrement im menschlichen Körper bei einem Treffer aufgrund des Impulsübertrags von der Stoßwelle eine makroskopische Bewegung ausführt. Bestrahlt man das Zielobjekt mit Ultraschallwellen und mißt die an ihm reflektierten Ultraschallwellen, so äußert sich diese makroskopische Bewegung in einer Dopplerverschiebung der Frequenz der reflektierten Wellen. Dementsprechend ausgestattete Lithotripter gemäß dem Oberbegriff des Anspruchs 1, bei denen die Auswertung der empfangenen Ultraschallwellen eine Doppleranalyse beinhaltet, sind beispielsweise aus der EP 0 367 116 B1, der EP 0 548 048 B1 und der DE 44 46 192 A1 bekannt. Diesen Geräten ist gemeinsam, daß der Ultraschall-Transducer Ultraschallwellen in den Körper aussendet und die vom Körper zum Ultraschall-Transducer zurückreflektierten Ultraschallwellen erfaßt, wobei eine Dopplersignaleinheit aus den ausgesandten und empfangenen Ultraschallwellen ein Dopplersignal generiert und auswertet, wobei im wesentlichen der Betrag einer Frequenzverschiebung des reflektierten Signals gegenüber den ausgesandten Wellen berechnet wird und hieraus auf die Treffgenauigkeit geschlossen wird.

Diese Vorgehensweise weist verschiedene Nachteile auf:

Es ist im Stand der Technik bereits bekannt, daß das Dopplersignal gerade in der Nähe seines zeitlichen Nullpunkts, d.h. unmittelbar nach dem Aussenden der Stoßwelle, Artefakte enthält. Um zu verhindern, daß genau solche Artefakte gemessen werden, weisen beispielsweise die Lithotripter gemäß der EP 0 367 116 B1 und der EP 0 548 048 B1 Mittel zur zeitlichen Synchronisierung zwischen dem Stoßwellengenerator und der Dopplersignaleinheit auf, was diese Geräte aufwendig und teuer macht.

Darüber hinaus kann es in der Umgebung von starken Streuern wie einem Konkrement im Dopplerspektrum zu dem sogenannten Spiegelartefakt kommen. Durch Zweifachreflektion wird die Bewegung eines Streuers zusätzlich in entgegengesetzer Richtung registriert. Dies führt zu einem zusätzlichen Betrag, der dem an der Nullinie gespiegelten Nutzsignal entspricht. Wegen der Kurzlebigkeit des Prozesses und der starken Artefakte können den Spektren nicht einfach Geschwindigkeits-Zeit-Verläufe und somit eine Trefferinformation zugeordnet werden.

US-A-5,287,856 beschreibt eine Vorrichtung zur Fokalbereichsortung für die Lithotripsie. Diese Vorrichtung umfasst eine Stoßwellenquelle sowie einen Ultraschallscanner zum Senden von Ultraschallwellen und einen Empfänger zum Empfangen deren Echos, die in einer Ultraschallvorrichtung zu einem Ultraschall-B-Bild des betrachteten Körperbereichs weiterverarbeitet werden. Diese B-Bilder werden in einer Vorrichtung zur Detektion der durch die Stoßwellen induzierten Bewegungsvorgänge weitergeleitet. Diese Vorrichtung umfasst einen Korrelator, der bestimmte Bereiche der B-Bilder korreliert und die Bildkoordinaten maximaler Korrelation für die Berechnung eines Bewegungsvektors verwendet.

US-A-4,819,621 beschreibt ein Verfahren zum Erkennen von möglichen Gewebeschädigungen. Diese möglichen Gewebeschädigungen können aufgrund von Kavitationen bei der medizinischen Anwendung von Hochenergie-Schall entstehen, der extern von einem Schallwandler erzeugt und über ein Koppelmedium in den Körper des Patienten übertragen wird. Dabei durchläuft das Schallfeld ein körperinneres Raumgebiet und wird auf einem Zielgebiet fokussiert, um ein im Zielgebiet befindliches Teil, wie etwa ein Konkrement oder einen Gewebeteil, zu zerstören

Es ist daher Aufgabe der vorliegenden Erfindung, einen Lithotripter der eingangs genannten Art bereitzustellen, der aus den empfangenen Ultraschallwellen eine Information im Hinblick auf die Treffer- und Desintegrationskontrolle ermittelt und zur Darstellung bringt, ohne hierbei die beschriebene apparativ aufwendige und fehleranfällige Doppleranalyse durchzuführen.

Erfindungsgemäß wird diese Aufgabe bei einem gattungsgemäßen Lithotripter dadurch gelöst, daß der Ultraschall-Transducer dazu ausgelegt ist, gepulste Ultraschallwellen auszusenden, und daß die Auswerteeinheit dazu ausgelegt ist, einen Koeffizienten der zeitlichen Korrelation zwischen reflektierten Ultraschallwellen zu bestimmen, die nacheinander ausgesandten Ultraschallpulsen zugeordnet sind, und ein zugeordnetes Korrelationskoeffizientensignal auszugeben.

Diese erfindungsgemäße Gestaltung des Lithotripters nutzt die Tatsache aus, daß die Korrelation zwischen nacheinander empfangenen "Echos", d.h. reflektierten Ultraschallwellen, um so größer ist, je statischer das reflektierende System ist, in diesem Fall also der Körperbereich, in dem das Zielgebiet des Stoßwellengenerators liegt und in dem die pulsweise ausgesandten Ultraschallwellen reflektiert werden.

Die folgende Überlegung mag dies veranschaulichen:

Wenn sich das reflektierende System, beispielsweise ein Nierenstein in einer Niere eines zu behandelnden Patienten, von einem ausgesandten Ultraschallpuls zum nächsten ausgesandten Ultraschallpuls nicht verändert hat, beispielsweise weil keine Stoßwelle vom Stoßwellengenerator ausgesandt wurde, oder weil eine solche Stoßwelle zwar ausgesandt wurde, aber zu einem "Fehlschuß" geführt hat, d.h. den Nierenstein verfehlt hat, so versteht es sich, daß das erste Echo, d.h. die Reflektion des ersten ausgesandten Ultraschallpulses, und das zweite Echo, also die Reflektion des zweiten ausgesandten Ultraschallpulses, weitgehend identisch sind. Bezeichnet man allgemein den zeitlichen Verlauf des i-ten empfangenen Ultraschallechos als $e_i(t)$ und entsprechend den zeitlichen Verlauf eines danach empfangenen Ultraschallechos, welches die Reflektion des (i+k)-ten ausgesandten Pulses darstellt, als $e_{i+k}(t)$, so kann man die zeitliche Korrelation zwischen diesen beiden reflektierten Ultraschallwellen definieren als:

$$K_{i,k} = \frac{\int_{T_1}^{T_2} e_i(t) e_{i+k}(t)\, dt}{\left(\int_{T_1}^{T_2} e_i^2(t)\, dt\right)^{1/2} \left(\int_{T_1}^{T_2} e_{i+k}^2(t)\, dt\right)^{1/2}} \tag{1}$$

Hierbei legen die untere Integrationsgrenze $T_1$ und die obere Integrationsgrenze $T_2$ das Zeitfenster fest, in dem die empfangenen Echosignale ausgewertet werden. $T_1$ und $T_2$ werden dabei so gewählt, daß das Echo aus dem Zielgebiet des Lithotripters stammt. Der Mittelwert dieses Zeitfensters $(T_1 + T_2)/2$ entspricht dabei dem Abstand zwischen dem Ultraschall-Transducer und dem Zielgebiet des Lithotripters. Andererseits bestimmt $(T_2 - T_1)/2$ die Größe des Volumens, das für die Auswertung herangezogen wird.

In der obigen Formel (1) stellt der Nenner eine Normierung des Korrelationskoeffizienten $K_{i,k}$ dar. Beim zunächst betrachteten Fall eines Fehlschusses, bei dem der reflektierende Körperbereich des Patienten weitgehend statisch bleibt, sind die Ultraschallechos $e_i(t)$ und $e_{i+k}(t)$ identisch, ihre Korrelation $K_{i,k}$ beträgt somit im wesentlichen 1. Aufgrund eines in der Praxis nie ganz vermeidbaren Rauschens werden jedoch $e_i(t)$ und $e_{i+k}(t)$ nicht exakt identisch sein, so daß in der Praxis stets $K_{i,k} < 1$ gilt.

Diese Situation ändert sich völlig, wenn anstelle eines Fehlschusses mit einer fokussierten Stoßwelle ein Treffer erzielt wird. Dies führt nämlich zu verschiedenen Effekten, die sich jeweils auf den zeitlichen Verlauf der empfangenen Ultraschallwellen auswirken: Ein Wechselwirkungsmechanismus zwischen der Stoßwelle und dem Konkrement führt zu einer makroskopischen Bewegung des Konkrements, insbesondere dann, wenn es bereits teilweise oder sogar größtenteils fragmentiert ist. Gerade am Anfang der Behandlung, wenn das Konkrement noch weitgehend unfragmentiert ist, dominieren andere Effekte, nämlich insbesondere das Herausschießen von Fragmenten aus dem Konkrement sowie das Auftreten von Kavitationsblasen um das Konkrement herum bei einem erfolgreichen Treffer. All diese Mechanismen führen zu einer dynamischen Bewegung von Bereichen im Körper des Patienten, an denen die ausgesandten Ultraschallpulse reflektiert werden und bewirken insgesamt, daß die Korrelation zwischen Ultraschallechos, die der Reflektion von nacheinander ausgesandten Ultraschallpulsen entsprechen, um so kleiner wird, je stärker die Wechselwirkung zwischen Stoßwelle und Konkrement ist und je stärker das Konkrement bereits fragmentiert ist. Somit enthält das von der Auswerteeinheit ausgegebene Korrelationskoeffizientensignal die gewünschten Informationen über die Stärke der Wechselwirkung zwischen der ausgesandten Stoßwelle und dem Zielobjekt.

Wie bereits der oben verwendeten Terminologie betreffend die zwei Ultraschallechos $e_i(t)$ und $e_{i+k}(t)$ zu entnehmen ist, können zur Bestimmung des zeitlichen Korrelationskoeffizienten zwei empfangene Ultraschallwellen verwendet werden, die zu ausgesandten Ultraschallpulsen gehören, die nicht unmittelbar nacheinander ausgesandt wurden, sondern durch weitere ausgesandte Ultraschallpulse getrennt sind. Vorzugsweise ist jedoch vorgesehen, daß die Auswerteeinheit dazu ausgelegt ist, den Korrelationskoeffizienten anhand von reflektierten Uitraschaliweilen zu bestimmen, die unmittelbar aufeinanderfolgenden ausgesandten Ultraschallpulsen zugeordnet sind. In der obigen Terminologie entspricht dies also dem Fall k = 1 und erlaubt die maximale Ausnutzung der von der Auswerteeinheit empfangenen Informationen. Betrachtet man in diesem Fall beispielsweise die ersten drei nach dem Erzeugen einer Stoßwelle ausgesandten Ultraschallpulse, so werden diese drei Ultraschallpulse im Zielgebiet des Stoßwellengenerators reflektiert und als Echos 1 bis 3 vom Ultraschall-Transducer empfangen und in der Auswerteeinheit ausgewertet, wobei diese die Korrelation zwischen dem ersten und dem zweiten Echo bestimmt, dann die Korrelation zwischen dem zweiten und dem dritten Echo usw. Im Anschluß an jede dieser Korrelationskoeffizientenberechnungen wird ein zugeordnetes Korrelationskoeffizientensignal von der Auswerteeinheit ausgegeben.

Hinsichtlich der Weiterverarbeitung der im Korrelationskoeffizientensignal enthaltenen Trefferinformation sind zahlreiche Vorgehensweisen möglich:

In einer bevorzugten Ausgestaltung des erfindungsgemäßen Lithotripters ist vorgesehen, daß er ferner eine mit der Auswerteeinheit verbundene Anzeigevorrichtung zur Anzeige des zeitlichen Verlaufs des Korrelationskoeffizienten aufweist. Die Korrelationskoeffizienten werden in einem Koordinatensystem dargestellt, dessen Abszisse die Zeit seit dem Aussenden der letzten Stoßwelle und deren Ordinate der ermittelte Korrelationskoeffizient ist. Bei einem Fehlschuß weist diese Kurve nahezu über die gesamte Zeit zwischen zwei aufeinanderfolgenden Stoßwellen einen konstanten Wert von etwa 1 auf. Bei einem Treffer hingegen zeigt sich aufgrund der beschriebenen Effekte beim Zielobjekt, welche die reflektierten Ultraschallechos beeinflussen, ein "Einbruch" im zeitlichen Verlauf des Korrelationskoeffizienten. Nach ca. 50 bis 100 ms kommen erfahrungsgemäß die Fragmente und das Konkrement selbst wieder zur Ruhe, und Kavitationsblasen sind nach diesem Zeitraum wieder verschwunden, so daß aufeinanderfolgende Ultraschallechos einander wieder ähnlicher werden. Der Korrelationskoeffizient relaxiert dann wieder in Richtung des Werts 1. Das den Lithotripter bedienende medizinische Personal kann also bereits anhand dieser erfindungsgemäß vorgesehenen Anzeige beurteilen, ob ein Fehlschuß oder ein Treffer vorliegt.

Zusätzlich oder alternativ kann vorgesehen sein, daß die Auswerteeinheit dazu ausgelegt ist, ein Fehlersignal auszugeben, wenn nach Aussenden einer Stoßwelle der Minimalwert des Korrelationskoeffizienten einen vorbestimmten ersten Schwellenwert nicht unterschreitet. Es ergibt sich nämlich aus der obigen Erläuterung der Auswirkungen eines Treffers auf den Korrelationskoeffizienten, daß dessen zeitlicher Verlauf einen um so stärkeren Einbruch aufweist, je besser der Treffer war, d.h. je stärker die Wechselwirkung zwischen der Stoßwelle und dem Zielobjekt war. Dementsprechend sollte der Einbruch beim Korrelationskoeffizienten bei einem guten Treffer stärker ausfallen, anders ausgedrückt kann ein Fehlschuß oder ein "Streifschuß" daran erkannt werden, daß in der oben geschilderten zeitaufgelösten Anzeige des Korrelationskoeffizienten sein Minimalwert den ersten Schwellenwert nicht unterschreitet. Die Ausgabe eines Fehlersignals in diesem Fall erlaubt es, diese Fehlschußinformation automatisch weiterzuverarbeiten, ohne auf ein Eingreifen des medizinischen Personals angewiesen zu sein.

Zusätzlich oder alternativ kann die Auswerteeinheit dazu ausgelegt sein, ein Fehlersignal auszugeben, wenn nach Aussenden einer Stoßwelle eine Relaxationszeit des Korrelationskoeffizienten einen vorbestimmten zweiten Schwellenwert unterschreitet. Ein Treffer äußert sich nämlich auch darin, daß das System aus Konkrement, Fragmenten und Kavitationsblasen eine bestimmte Mindestzeit benötigt, um wieder zur Ruhe zu gelangen, entsprechend einer Mindestrelaxationszeit des Korrelationskoeffizienten, d.h. der Breite des Einbruchs bei der zeitlichen Auftragung des Korrelationskoeffizienten. Insbesondere kann im Verlauf der gesamten Behandlung die Relaxationszeit in Form einer fortlaufenden Anzeige dargestellt werden, um die Entwicklung der Relaxationszeit zu verfolgen. Mit erfolgreicher fortschreitender Zertrümmerung des Zielobjekts sollte die Relaxationszeit des Korrelationskoeffizienten kontinuierlich zunehmen. Nimmt die Relaxationszeit jedoch ab, so ist dies ein starkes Indiz dafür, daß sich das Konkrement aus dem Stoßwellenfokus herausbewegt hat, sei es durch eine Konkrementbewegung (beispielsweise die Bewegung eines Nierensteins innerhalb der Niere des Patienten) oder durch eine Verschiebung des Patienten selbst. Als Anzeigevorrichtung kann hierbei gegebenenfalls der Bildschirm verwendet werden, auf dem auch die von der bildgebenden Ortungsvorrichtung gelieferten Bilder dargestellt werden.

Die Ermittlung der Relaxationszeit kann auf verschiedene Weisen erfolgen. Beispielsweise kann die Auswerteeinheit dazu ausgelegt sein, die Relaxationszeit durch Anpassen einer Gauss-Kurve an den zeitlichen Verlauf des Korrelationskoeffizienten zu bestimmen. Je nach in der Praxis auftretendem Kurvenverlauf kann es auch zweckmäßig sein, eine Funktion der Form $1 - A\exp(-t/T_R)$ an den zeitlichen Verlauf des Korrelationskoeffizienten anzupassen, wobei $T_R$ die durch Anpassung zu ermittelnde Relaxationszeit ist. Ebenso ist es in einer besonders einfachen Ausführungsform möglich, die Relaxationszeit $T_R$ über Schwellenwerte zu definieren, d. h. als die Differenz derjenigen Zeitpunkte zu bestimmen, an denen der zeitliche Verlauf des Korrelationskoeffizienten erstmals unter einen bestimmten Schwellenwert sinkt bzw. in der Relaxationsphase dann wieder über diesen Schwellenwert ansteigt.

Derartige Kurvenanpassungen ("Fitten") und Schwellenwert-Analysen können heute von zahlreichen Datenverarbeitungssystemen schnell und sicher durchgeführt werden, so daß eine Echtzeitkontrolle der Stoßwellentherapie mit Hilfe der Auswerteeinheit möglich ist.

In einer vorteilhaften Weiterbildung der Erfindung kann der erfindungsgemäße Lithotripter eine mit der Auswerteeinheit verbundene Alarmvorrichtung aufweisen, der das Fehlersignal zugeführt wird, wobei in diesem Fall zweckmäßigerweise vorgesehen ist, daß die Alarmvorrichtung zur Abgabe eines optischen und/oder akustischen Alarms ausgebildet ist. Somit kann die erfindungsgemäß vorgeschlagene Alarmvorrichtung beispielsweise einen Warnton aussenden oder eine Warnleuchte einschalten, sobald sich aus dem zugeführten Fehlersignal ergibt, daß der Minimalwert des Korrelationskoeffizienten den ersten Schwellenwert nicht unterschreitet bzw. daß seine Relaxationszeit den zweiten Schwellenwert unterschreitet, was jeweils auf einen Fehlschuß hinweist.

Zusätzlich oder alternativ dazu kann der Alarm dann ausgelöst werden, wenn die gemessenen Relaxationszeiten einen negativen Trend aufweisen. Bei einer Desintegration ist nämlich von einer steigenden Relaxationszeit auszugehen.

Alternativ oder zusätzlich kann vorgesehen sein, daß der Stoßwellengenerator mit der Auswerteeinheit verbunden und dazu ausgelegt ist, die Erzeugung von Stoßwellen als Funktion des Fehlersignals zu stoppen bzw. fortzusetzen. Hierdurch läßt sich eine automatische Abschaltung des Stoßwellengenerators erreichen, wenn offenbar das Zielobjekt nicht mehr im Stoßwellenfokus liegt. Somit wird unabhängig von der Aufmerksamkeit oder der Reaktionszeit des den Lithotripter bedienenden medizinischen Personals eine unnötige Belastung des Körpers des Patienten durch Stoßwellen-Fehlschüsse vermieden.

Die oben genannten Schwellenwerte, deren Über- bzw. Unterschreiten dazu führt, daß die Auswerteeinheit Fehlersignale ausgibt, können grundsätzlich in der Auswerteeinheit voreingestellt sein und hierbei sowohl die technischen Daten des Stoßwellengenerators (insbesondere seine Leistung) als auch typische Patientendaten berücksichtigen. Zweckmäßigerweise ist jedoch vorgesehen, daß die Auswerteeinheit Einstellmittel zum Einstellen des ersten und/oder des zweiten Schwellenwerts aufweist. Dann können diese Schwellenwerte zu Beginn der Behandlung auch unter Berücksichtigung des auftretenden Signalrauschens individuell eingestellt werden. Die Schwellenwerte können jedoch auch automatisch aus dem Signal selbst bestimmt werden. Aus den vor dem Beginn der Stoßwellentherapie akquirierten Signalen läßt sich nämlich ein idealer Verlauf des Korrelationskoeffizienten ebenso wie ein Grundrauschen bestimmen.

In einer Weiterbildung des erfindungsgemäßen Lithotripters kann die Auswerteeinheit dazu ausgelegt sein, den Verlauf der Werte des Korrelationskoeffizienten zu glätten, insbesondere durch Mittelung. Die Zahl der Korrelationskoeffizientenwerte, über die gemittelt wird, sollte hierbei vorzugsweise variabel wählbar sein.

Auf diese Weise wird verhindert, daß beispielsweise ein einzelner "Ausreißer", der z.B. durch ein numerisches Artefakt beim Berechnen eines Korrelationskoeffizienten verursacht sein kann, zur Ausgabe des Fehlersignals durch die Auswerteeinheit führt. Ebenso kann eine Synchronisation zwischen dem Ultraschall-Transducer und der Stoßwellenquelle vorgesehen sein, um zu verhindern, daß elektromagnetische Störungen, die durch die Betätigung der Stoßwellenquelle verursacht werden, eines der in diesem Augenblick empfangenen Echos stark stören, was zu einem Ausreißer im Korrelationskoeffizienten führen könnte.

Ebenso kann die Auswerteeinheit ferner dazu ausgelegt sein, den Minimalwert und/oder die Relaxationszeit des Korrelationskoeffizienten über mehrere Stoßwellen zu mitteln. Hierbei sind nämlich die folgenden geometrischen Umstände einer typischen Lithotripsie-Behandlung zu berücksichtigen: Der Stoßwellenfokus hat typischerweise eine Ausdehnung von ca. 4 mm. Ein typisches Konkrement, beispielsweise ein Nierenstein, weist am Anfang der Behandlung Abmessungen zwischen 5 und 20 mm auf, und seine Hin- und Herverlagerung durch die reine Atmung des Patienten kann mit einer Amplitude von ca. 30 mm erfolgen. Unter diesen Bedingungen würden, da das Aussenden von Stoßwellen typischerweise ohne Berücksichtigung des jeweiligen Atmungszustands des Patienten erfolgt, zwangsläufig einige Stoßwellen das Konkrement verfehlen. Die im unmittelbaren Anschluß an derartige Stoßwellen gemessenen Minimalwerte des Korrelationskoeffizienten sind - wie oben erläutert - besonders groß, im Extremfall hat der Korrelationskoeffizient den konstanten Wert 1. Entsprechend sind die im unmittelbaren Anschluß an derartige Stoßwellen gemessenen Relaxationszeiten besonders kurz, so daß unabhängig davon, welcher dieser beiden Parameter der Ausgabe eines Fehlersignals zugrunde gelegt wird, stets die Alarmvorrichtung betätigt und/oder der Stoßwellengenerator abgeschaltet würde, obwohl, keinerlei Dejustage des Patienten gegeben ist. Vielmehr würden bereits die nächsten Stoßwellen wieder das Konkrement treffen, da sie bei einem anderen Atmungszustand des Patienten ausgesandt werden. Bei der in dieser erfindungsgemäßen Ausführungsform vorgesehenen Mittelung der Minimalwerte bzw. der Relaxationszeiten über mehrere Stoßwellen kann zunächst in einer Ersteinstellung der Auswerteeinheit eine Mittelung über beispielsweise fünf Stoßwellen erfolgen, es empfiehlt sich jedoch zu weiterer Steigerung der Behandlungseffizienz, die Zahl der Stoßwellen, über die gemittelt wird, zu Beginn der Behandlung des Patienten abhängig von seinem typischen Atmungsverhalten, von der Größe des zu fragmentierenden Konkrements etc. individuell einzustellen. Grundsätzlich sind anstatt einer Glättung bzw. Mittelung auch andere Arten der Signalfilterung möglich, z.B. eine Medianfilterung.

Bei denjenigen Versionen von gattungsgemäßen Lithotriptern, die Doppleranalysen durchführen, hat sich in klinischen Experimenten eine deutliche Abhängigkeit zahlreicher Ultraschallmessungen von individuellen Patienten gezeigt. Daher kann die Auswerteeinheit in einer zweckmäßigen Weiterbildung des erfindungsgemäßen Lithotripters dazu ausgelegt sein, den Minimalwert und/oder die Relaxationszeit des Korrelationskoeffizienten auf den Referenz-Minimalwert bzw. die Referenz-Relaxationszeit einer Referenz-Korrelationskoeffizientenkurve zu normieren. Diese Referenz - Korrelationskoeffizientenkurve wird zweckmäßigerweise zu Beginn der Behandlung aufgenommen, wenn der Patient in der oben beschriebenen Weise derart im Lithotripter justiert worden ist, daß - wie sich anhand der bildgebenden Ortungsvorrichtung überwachen läßt - beispielsweise ein Nierenstein exakt im Stoßwellenfokus liegt. Bei der obengenannten Verlaufsanzeige, d.h. der fortlaufenden Auftragung beispielsweise von Relaxationszeiten im Verlauf der Stoßwellenbehandlung, werden dann also keine absoluten Relaxationszeiten des Korrelationskoeffizienten aufgetragen, sondern normierte Relaxationszeiten.

Bei der bislang beschriebenen Vorgehensweise wird der Korrelationskoeffizient gemäß der obigen Formel (1) berechnet. Sie führt dazu, daß eine Fragmentierung des Zielobjekts einen Einbruch im zeitlichen Verlauf des Korrelationskoeffizienten bewirkt, der gemäß einer charakteristischen Relaxationszeit in Richtung des Werts 1 relaxiert, wenn das System aus Zielobjekt, Fragmenten und Kavitationsblasen nach dem Treffer wieder zur Ruhe gekommen ist. Allerdings führt die Formel (1) auch dann zu einer Abnahme des Korrelationskoeffizienten, wenn sich zwischen dem Aussenden des i-ten Ultraschallpulses und des (i+k)-ten Ultraschallpulses beispielsweise das Konkrement aufgrund der Atmungsbewegung des Patienten ein wenig verschoben hat. Die gemäß Formel (1) arbeitende Auswerteeinheit würde dann bereits eine Abnahme des Korrelationskoeffizienten berechnen, da nämlich aufgrund der Verschiebung des Zielobjekts der zeitliche Verlauf $e_{i+k}(t)$ des (i+k)-ten empfangenen Ultraschallechos auf der zeitlichen Abszisse gegenüber dem Verlauf $e_i(t)$ des i-ten Ultraschallechos verschoben ist. Dies ist darauf zurückzuführen, daß beispielsweise bei einer geringfügigen Bewegung des Konkrements vom Ultraschall-Transducer weg die (i+k)-te ausgesandte Ultraschallwelle eine etwas längere Strecke durchlaufen muß als die i-te Welle, bevor sie reflektiert wird, und daß somit zwangsläufig auch das (i+k)-te Echo die gleiche zusätzliche Strecke zurücklaufen muß. Um derartige Einflüsse auf die berechneten Korrelationskoeffizienten zu eliminieren, kann in einer präziseren Ausführungsform des erfindungsgemäßen Lithotripters vorgesehen sein, daß die Auswerteeinheit dazu ausgelegt ist, eine zeitliche Kreuzkorrelationsfunktion zwischen reflektierten Ultraschallwellen zu ermitteln und als Korrelationskoeffizienten den Maximalwert der zeitlichen Kreuzkorrelationsfunktion zu bestimmen. In diesem Fall berechnet die Auswerteeinheit den Korrelationskoeffizienten im wesentlichen gemäß der nachfolgenden Formel:

$$K_{i,k} = \max_{\Delta t} \left( \int_{T1}^{T2} e_i(t) e_{i+k}(t - \Delta t) dt \right) \qquad (2),$$

wobei der Ausdruck in der Klammer die Kreuzkorrelationsfunktion mit der Variablen $\Delta t$ darstellt.

Bei dieser Ausführungsform verschiebt also die Auswerteeinheit den gemessenen Verlauf des (i+k)-ten Ultraschallechos entlang der Zeit-Abszisse jeweils um einen Betrag $\Delta t$ und bildet erst dann ähnlich Formel (1) das zeitliche Integral. Die auf diese Weise für zahlreiche Werte von $\Delta t$ berechneten Korrelationskoeffizienten werden zwischengespeichert, ihr Maximum wird schließlich als gesuchter Korrelationskoeffizient $K_{i,k}$ bestimmt. Dieser Algorithmus ist als Kreuzkorrelationsverfahren bekannt und wird im medizinischen Bereich beispielsweise bei der Bestimmung von Blutflußgeschwindigkeitsprofilen eingesetzt, wobei im wesentlichen derjenige Wert von $\Delta t$ gesucht wird, der das Maximum von K ergibt. Es wird in diesem Zusammenhang hingewiesen auf die Veröffentlichung "Flow Velocity Profile via Time-Domain Correlation: Error Analysis and Computer Simulation" von Steven G. Foster, Paul M. Embree, William D. O'Brien jr., IEEE Transactions on Ultrasonics, Ferroelectrics and Frequency Control, Band 37, Nr. 2, Mai 1990, Seite 164, sowie auf die Veröffentlichung "Time Domain Formulation of Pulse-Doppler Ultrasound and Blood Velocity Estimation by Cross Correlation" von O. Bonnefous und P. Pesque', Ultrasonic Imaging 8, 1986, Seiten 73 bis 85.

Im Hinblick auf mögliche medizinische Informationen, die in $\Delta$t enthalten sein können, kann selbstverständlich auch das Zwischenspeichern dieses Meßparameters vorgesehen sein.

Zweckmäßigerweise ist bei allen Ausführungsformen der Ultraschall-Transducer der Ultraschall-Sende-/Empfangseinheit an einem einstellbaren Halter montiert. Der Ultraschall-Transducer kann dann unabhängig von anderen Teilen des Lithotripters zur Optimierung der empfangenen Ultraschallsignale justiert und in der optimalen Position festgestellt werden. Insbesondere kann hierdurch gewährleistet werden, daß der Ultraschall-Transducer auf den Stoßwellenfokus gerichtet ist. Alternativ zu dieser sogenannten isozentrischen Scannerführung kann auch ein sogenannter Inline-Transducer eingesetzt werden, d.h. ein Ultraschall-Transducer, der in die Stoßwellenquelle integriert ist.

Grundsätzlich ist hierbei folgendes zu beachten: Durch den einstellbaren Halter wird lediglich eine Linie festgelegt, entlang der sich bei einem PW-Verfahren (pulse wave) ein ausgesandter Ultraschallpuls im Gewebe ausbreitet und Echos generiert werden. Bei der isozentrischen Bauweise ist dann gewährleistet, daß sich der Stoßwellenfokus, d.h. das Zielgebiet, auf dieser Linie befindet. Durch bekannte Mittel, beispielsweise einen Weggeber, läßt sich der Abstand des Transducers zum Fokus bestimmen. Aufgrund der bekannten Laufzeit von Ultraschallpulsen im Gewebe läßt sich aus diesem Abstand wiederum ein Zeitfenster definieren, das denjenigen Anteil des Echos herausschneidet, der im Zielgebiet generiert wurde, nämlich das Zeitfenster ($T_1$, $T_2$) gemäß Formel (1).

In einer Ausführungsform des erfindungsgemäßen Lithotripters kann vorgesehen sein, daß die Ultraschall-Sende/Empfangseinheit als Teil eines bildgebenden Ultraschallscanners ausgebildet ist (Duplex-Scanner). In diesem Fall können insbesondere der Ultraschall-Transducer und Teile der Elektronik der Ultraschall-Sende/Empfangseinheit gleichzeitig für die Ultraschall-Bildgebung und für die Messung der Ultraschallechopulse eingesetzt werden.

Alternativ kann der Ultraschall-Transducer als preisgünstige Stiftsonde ausgebildet sein, was ihn bei Verwendung mit dem oben genannten Halter flexibel einsetzbar macht, um beispielsweise sicherzustellen, daß er immer auf den Stoßwellenfokus gerichtet ist.

Insbesondere empfiehlt sich eine derartige Gestaltung, wenn der erfindungsgemäße Lithotripter ferner eine Röntgenortungsvorrichtung aufweist. In diesem Fall erfolgt die Bildgebung zu Beginn der Positionierung des Patienten sowie bei der etwa alle drei bis fünf Minuten erfolgenden Detailkontrolle mit Hilfe dieser bildgebenden Röntgenortungsvorrichtung, während die kontinuierliche Trefferkontrolle mit Hilfe des als Stiftsonde gestalteten Ultraschall-Transducers erfolgt. Es versteht sich, daß auch in diesem Fall die Anzeige der Korrelationskoeffizienten auf der auch für die Anzeige der Röntgenbilder eingesetzten Anzeigevorrichtung erfolgen kann.

Vorzugsweise ist beim erfindungsgemäßen Lithotripter ferner vorgesehen, daß er Mittel zur fortlaufenden Darstellung des Minimalwerts und/oder der Relaxationszeit des Korrelationskoeffizienten über den Behandlungsverlauf umfaßt. Auch hierbei kann es sich um die oben bereits genannte Anzeigevorrichtung handeln. Das den Lithotripter bedienende medizinische Personal erhält somit einen umfassenden Überblick über den gesamten Behandlungsverlauf.

Die vorliegende Erfindung betrifft ferner ein allgemeines Verfahren zur Überwachung der Fragmentierung eines Zielobjekts, insbesondere eines Konkrements, in einem vorzugsweise menschlichen Körper, umfassend die Schritte: Aussenden von Ultraschallwellen in den Körper, Empfangen von am Zielobjekt reflektierten Ultraschallwellen, und Auswerten der empfangenen Ultraschallwellen, welches dadurch gekennzeichnet ist, daß im Schritt des Aussendens von Ultraschallwellen gepulste Ultraschallwellen ausgesandt werden, und daß der Schritt des Auswertens eine Bestimmung eines Koeffizienten der zeitlichen Korrelation zwischen reflektierten Ultraschallwellen umfaßt, die nacheinander ausgesandten Ultraschallpulsen zugeordnet sind, und daß ferner ein Schritt der Ausgabe eines zugeordneten Korrelationskoeffizientensignals vorgesehen ist.

Wie oben erläutert kann dieses Verfahren im Fall seiner Anwendung bei Lithotripsie derart weiter entwickelt werden, daß es ferner die Schritte umfaßt: Anordnen des Körpers in einem Lithotripter mit einem Stoßwellengenerator zur Erzeugung fokussierter Stoßwellen, Anzeigen des Zielobjekts und des Fokus des Stoßwellengenerators auf einer Anzeigevorrichtung einer bildgebenden Ortungsvorrichtung, Justieren des Körpers derart, daß das Zielobjekt im Fokus des Stoßwellengenerators liegt, Aussenden einer Stoßwelle in Richtung des Zielobjekts und Bestimmen des Minimalwerts und/oder der Relaxationszeit des Korrelationskoeffizienten, Speichern des Minimalwerts bzw. der Relaxationszeit als Referenz-Minimalwert bzw. Referenz-Relaxationszeit, sowie den späteren Schritt: Normieren des Minimalwerts und/oder der Relaxationszeit eines später gemessenen Korrelationskoeffizienten auf den Referenz-Minimalwert bzw. die Referenz-Relaxationszeit. Auf diese Weise wird eine Normierung der gemessenen Minimalwerte bzw. Relaxationszeiten des Korrelationskoeffizienten auf für diesen Patienten und für dieses Konkrement typische Werte erreicht.

Zweckmäßigerweise kann aus den oben genannten Gründen eine fortlaufende Darstellung des Minimalwerts und/oder der Relaxationszeit des Korrelationskoeffizienten über den Behandlungsverlauf vorgesehen sein.

Ein Ausführungsbeispiel der Erfindung wird im folgenden anhand der Zeichnung erläutert werden. Es zeigen:

Fig. 1    eine vereinfachte Darstellung wesentlicher Bauteile des erfindungsgemäßen Lithotripters;

Fig. 2    eine schematische Ansicht der Auswerteeinheit und ihrer Anschlüsse beim erfindungsgemäßen Lithotripter;

Fig. 3    ein Beispiel zweier nacheinander empfangener Ultraschallwellenechos, die an einem statischen System bei einem Fehlschuß der Stoßwelle reflektiert wurden;

Fig. 4    ein Beispiel zweier nacheinander empfangener Ultraschallwellenechos, die an einem in Bewegung befindlichen System bei einem Treffer der Stoßwelle reflektiert wurden;

Fig. 5    ein schematisches Beispiel für den zeitlichen Verlauf des Korrelationskoeffizienten bei einem Treffer einer Stoßwelle;

Fig. 6    ein schematisches Flußdiagramm zur Verdeutlichung der Hauptabschnitte des erfindungsgemäßen Verfahrens;

Fig. 7    ein schematisches Flußdiagramm zur Verdeutlichung wesentlicher Verfahrensschritte innerhalb des ersten Hauptabschnitts gemäß Fig. 6;

Fig. 8    ein schematisches Flußdiagramm zur Verdeutlichung wesentlicher Verfahrensschritte innerhalb des zweiten Hauptabschnitts gemäß Fig. 6;

Fig. 9    ein schematisches Flußdiagramm zur Verdeutlichung wesentlicher Verfahrensschritte des dritten Hauptabschnitts gemäß Fig. 6; und

Fig. 10   ein schematisches Beispiel für eine Relaxationszeit-Verlaufskontrolle während einer Stoßwellentherapie.

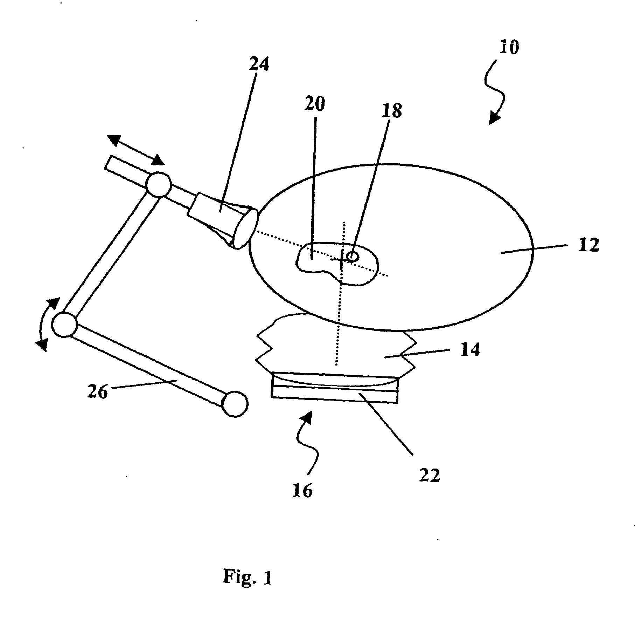

Fig. 1 zeigt eine schematische Ansicht wesentlicher mechanischer Komponenten des erfindungsgemäßen Lithotripters 10. Ein Patient 12 ist derart auf einer in Fig. 1 nicht dargestellten verstellbaren Liege gelagert, daß ein Ankoppelkissen 14 eines Stoßwellengenerators 16 an der gewünschten Stelle an den Körper des Patienten 12 gedrückt werden kann, um Stoßwellen in Richtung eines im Körper des Patienten 12 zu fragmentierenden Konkrements 18 auszusenden. In dem in Fig. 1 schematisch dargestellten Fall ist dieses Konkrement 18 ein Nierenstein in der Niere 20 des Patienten 12. Der Patient 12 wird mit Hilfe der verstellbaren Liege derart "einjustiert", d.h. positioniert, daß der in Fig. 1 durch ein Kreuz angedeutete Fokus der Stoßwellen, die von einer Stoßwellenquelle 22 des Stoßwellengenerators 16 erzeugt und mit Hilfe des Ankoppelkissens 14 in den Körper des Patienten 12 hineingesendet werden, im Nierenstein 18 liegt. Diese Justage erfolgt üblicherweise mit Hilfe einer bildgebenden Ortungsvorrichtung, beispielsweise einer Röntgenvorrichtung oder eines Ultraschallscanners.

Diese Komponenten des Lithotripters 10 und diese Vorgehensweise bei der Positionierung des Patienten vor Beginn der Behandlung sind an sich bekannt und werden hier nicht näher erläutert werden.

Der erfindungsgemäße Lithotripter 10 umfaßt ferner einen Ultraschall-Transducer 24, der an einem einstellbaren Halter 26 montiert ist. Bei dem in Fig. 1 gezeigten Beispiel ist dieser Halter 26 in Form eines gelenkigen Arms ausgebildet und ermöglicht eine genaue Positionierung des Ultraschall-Transducers 24 an einer gewünschten Stelle des Körpers des Patienten 12 derart, daß der Ultraschall-Transducer 24 - wie in Fig. 1 durch gepunktete Linien angedeutet - auf den Stoßwellenfokus gerichtet ist. Man spricht bei dieser Anordnung von einer isozentrischen Scannerführung.

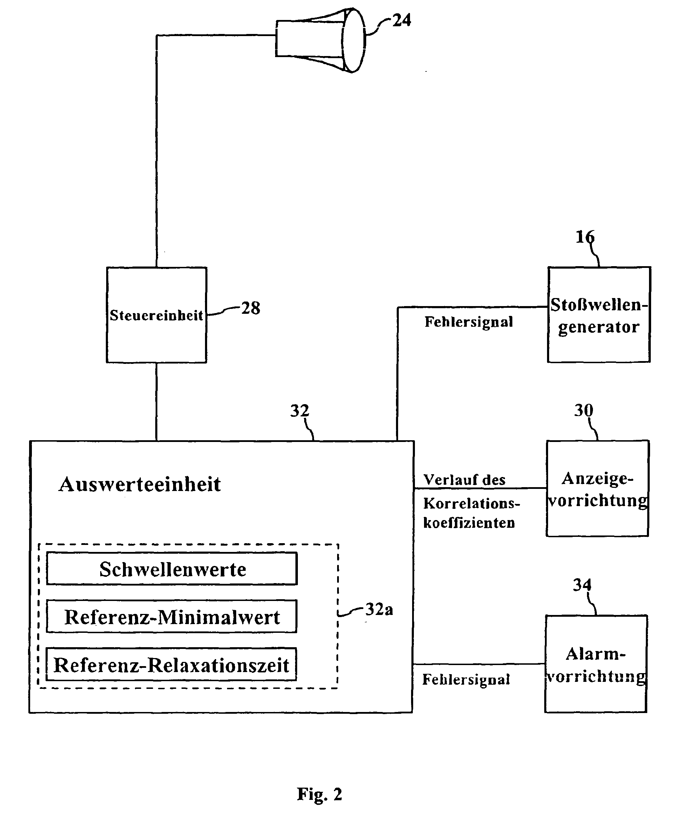

Der Ultraschall-Transducer 24 sendet in Pulsen (pulsed wave, PW) Ultraschallwellen in Richtung des Stoßwellenfokus aus und empfängt ferner Ultraschallwellen, die im Körper des Patienten 12, insbesondere aus dem Gebiet des Stoßwellenfokus, reflektiert worden sind. Die empfangenen Ultraschallsignale werden, wie in Fig. 2 angedeutet, vom Ultraschall-Transducer 24 einer Steuereinheit 28 zugeführt, die nicht nur als Teil einer Sende-/Empfangseinheit den eigentlichen Betrieb des Ultraschall-Transducers 24 steuert, beispielsweise die in ihm enthaltenen piezoelektrischen Elemente, und die vom Ultraschall-Transducer 24 gemessenen reflektierten Ultraschallsignale erfaßt, sondern diese auch an nachgeordnete Elektronikeinheiten weiterleitet. Beispielsweise könnte die Steuereinheit 28 die empfangenen Ultraschallsignale an ein in Fig. 2 nicht dargestelltes Bildverarbeitungsmodul weiterleiten, mit dessen Hilfe auf einer Anzeigevorrichtung 30 Ultraschallbilder des Nierensteins dargestellt werden können.

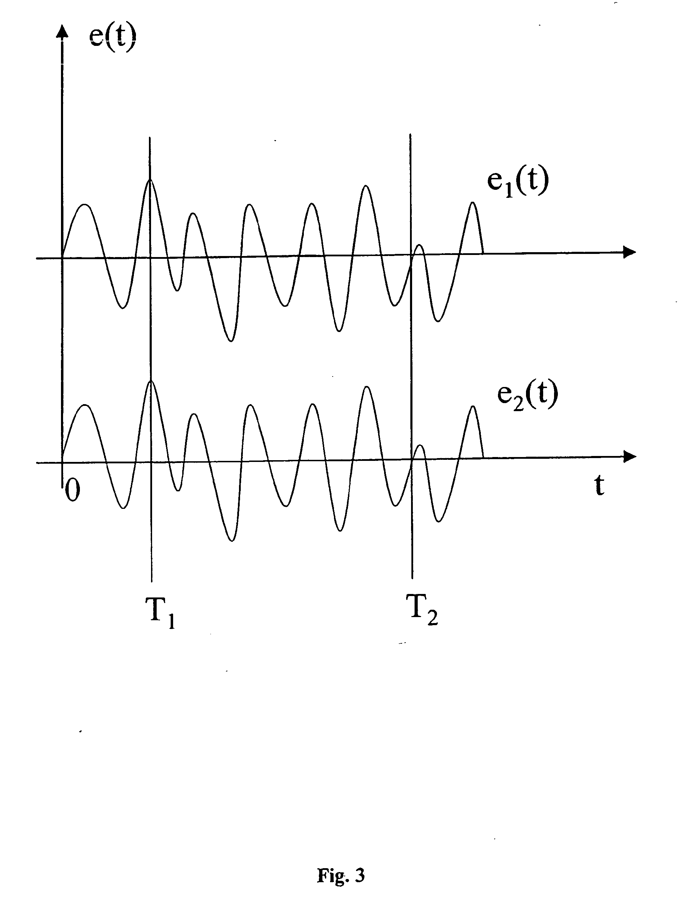

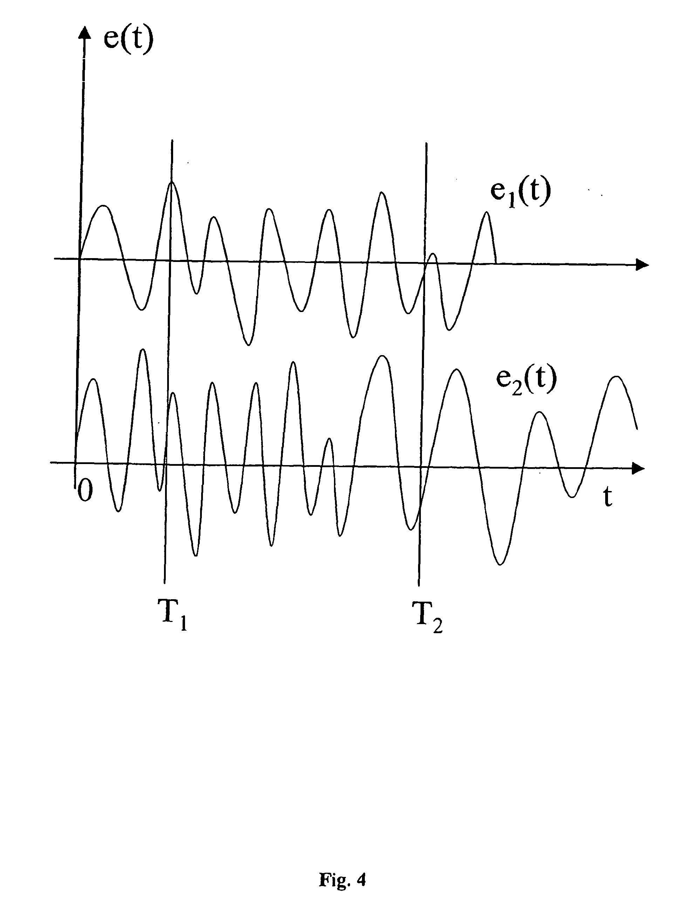

Unabhängig hiervon führt die Steuereinheit 28 beim erfindungsgemäßen Lithotripter 10 die empfangenen Ultraschallsignale einer Auswerteeinheit 32 zu, die derart gebildet ist, daß sie einen zeitlichen Korrelationskoeffizienten zwischen reflektierten Ultraschallwellen bestimmt, die nacheinander ausgesandten Ultraschallpulsen zugeordnet sind. Dies wird im folgenden anhand der Fig. 3 und 4 erläutert werden:

Der Ultraschall-Transducer 24 sendet mit einer Frequenz von typischerweise etwa 1 kHz Ultraschallpulse aus, die im Körper des Patienten 12 reflektiert werden, und zwar aufgrund der vorherigen Justage des Ultraschall-Transducers 24 mit Hilfe des einstellbaren Halters 26 in einem Zielgebiet des Stoßwellengenerators 16. Da der Stoßwellengenerator 16 etwa eine Stoßwelle pro Sekunde aussendet, bedeutet dies, daß zwischen zwei Stoßwellen etwa 1000 Ultraschallpulse in den Körper des Patienten 12 ausgesandt und demzufolge auch ca. 1000 Ultraschallechos empfangen werden. Diese empfangenen Ultraschallechos werden allgemein als $e_1, e_2 ... e_{1000}$ bezeichnet. Fig. 3 zeigt rein beispielhaft den zeitaufgelösten Verlauf zweier unmittelbar nacheinander empfangener Ultraschallechos $e_1(t)$ und $e_2(t)$, wobei zu beachten ist, daß die Auswerteeinheit 32 einen internen Zeitzähler bei jedem Aussenden eines Ultraschallpulses wieder auf 0 zurücksetzt. Fig. 3 zeigt den Fall, bei dem das Zielgebiet des Stoßwellengenerators 16, in dem die ausgesandten Ultraschallwellen reflektiert werden, völlig statisch ist, etwa deswegen, weil momentan gar keine Stoßwellen ausgesandt werden, oder auch deswegen, weil ausgesandte Stoßwellen das Konkrement 18 verfehlen. Das Konkrement 18 bleibt somit in Ruhe, es folgen keinerlei Umschichtungen oder Fragmentierungen des Konkrements 18, und es treten auch keine Kavitationsblasen in der unmittelbaren Umgebung des Konkrements 18 auf. Dementsprechend sind die zeitlichen Verläufe der beiden betrachteten Ultraschallechos $e_1(t)$ und $e_2(t)$ nahezu identisch, so daß die gemäß der oben angegebenen Formel (1) durch Aufintegrieren des Produkts $e_1(t) \cdot e_2(t)$ über das Zeitfenster von $T_1$ bis $T_2$ berechnete Korrelation maximal ist und bei geeigneter Skalierung gemäß der obigen Formel (1) etwa den Wert 1 annimmt.

Fig. 4 zeigt einen Fall, bei dem das Ultraschallecho $e_1$ noch an dem gleichen ruhenden Zielgebiet reflektiert wurde, das Konkrement 18 jedoch unmittelbar danach von einer Stoßwelle getroffen wurde. Liegt das Konkrement 18 im Stoßwellenfokus, so daß beim Aussenden der Stoßwelle ein Treffer erzielt wird, so werden kleine Fragmente aus dem Konkrement 18 herausgeschlagen und gleichzeitig treten im Konkrement 18 Umschichtungen auf. In der unmittelbaren Umgebung des Konkrements 18 kommt es ferner in der körpereigenen Flüssigkeit des Patienten 12 zu erhöhter Kavitation. All diese Prozesse führen dazu, daß die vom Ultraschall-Transducer 24 in Richtung des Konkrements 18 ausgesandten Ultraschallwellen zunehmend an Objekten reflektiert werden, die gegenüber dem Transducer 24 nicht nur an einer anderen Position als noch unmittelbar zuvor bei der Reflektion des Echos $e_1$ sind, sondern zudem auch gegenüber dem Transducer 24 in Bewegung sind. Die reine Tatsache, daß sich bestimmte reflektierende Bereiche, z.B. das Konkrement 18 selbst, bezogen auf die unmittelbar vorangegangene Reflektion bewegt haben, beispielsweise vom Transducer 24 weg, führt zu veränderten Signallaufzeiten und somit zu einer Verschiebung der Kurve $e_2(t)$ relativ zur Kurve $e_1(t)$. Zudem führt die Tatsache, daß aufgrund des Treffers bestimmte reflektierende Bereiche nunmehr nicht mehr statisch, sondern in Bewegung sind, zum Auftreten von Änderungen des Signalverlaufs im reflektierten Echo $e_2$, d. h. zu komplexen Änderungen der Signal-Zeit-Funktion. Auch die bei einem Treffer in der unmittelbaren Umgebung des Konkrements 18 verursachte erhöhte Kavitation führt zu einer ungeregelten Bewegung von reflektierenden Objekten (Dichteschwankungen bzw. Gasblasen in der Körperflüssigkeit des Patienten 12), was ebenso zu Änderungen des Signalverlaufs führt.

Auch eine makroskopische Bewegung des Konkrements 18, die insbesondere dann auftreten kann, wenn es bereits mindestens teilweise fragmentiert ist, führt sowohl zu einer Veränderung von Signallaufzeiten als auch zu einer komplexen Änderung der Signal-Zeit-Funktion, wenn das Konkrement 18 aufgrund von elastischen Kräften des umliegenden Gewebes eine Art Schwingungsbewegung vollführt.

Man versteht daher bei Betrachtung der Fig. 4 sofort, daß der wiederum gemäß der obigen Formel (1) berechnete Korrelationskoeffizient zwischen den zeitlichen Verläufen $e_1(t)$ und $e_2(t)$ kleiner sein wird als bei dem in Fig. 3 gezeigten Beispiel weitgehend identischer Echos, die an einem statischen System reflektiert werden.

Die Auswerteeinheit 32 berechnet fortlaufend die Korrelationskoeffizienten zwischen $e_1$ und $e_2$, zwischen $e_2$ und $e_3$, zwischen $e_3$ und $e_4$ usw., die aufgrund der oben beschriebenen Mechanismen im Fall eines Stoßwellentreffers am Konkrement 18 fortlaufend kleiner werden. Erfahrungsgemäß kommen nach einem derartigen Treffer die Fragmente und das Konkrement 18 selbst nach typischerweise 50 bis 100 ms wieder zur Ruhe, die Kavitationsblasen sind nach Ablauf dieser Zeit ebenfalls wieder verschwunden, so daß aufeinanderfolgende Echos $e_i, e_{i+1}$ einander wieder ähnlicher werden und der von der Auswerteeinheit 32 berechnete Korrelationskoeffizient somit wieder in Richtung seines Maximalwerts 1 relaxiert, d.h. ansteigt. Wie oben bereits erläutert, ist insbesondere davon auszugehen, daß der Verlauf des Korrelationskoeffizienten durch eine Funktion der Form $1 - A \exp(-t/T_R)$ beschrieben wird, je nach individueller Situation kann der Kurvenverlauf aber auch zumindest abschnittsweise einer Gausskurve ähneln.

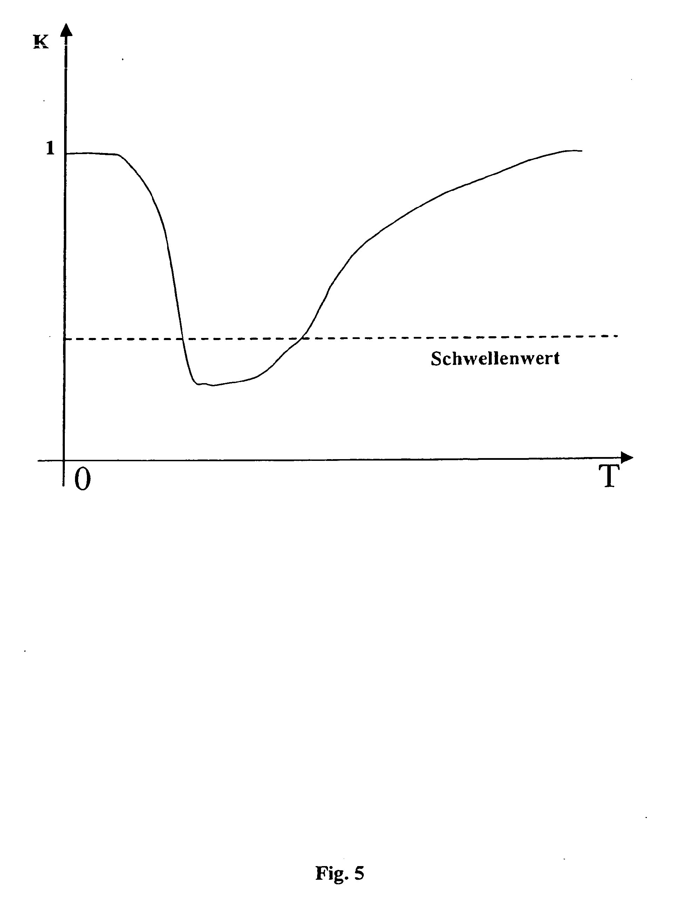

Der somit zu erwartende Verlauf des Korrelationskoeffizienten K als Funktion der Zeit ist in Fig. 5 dargestellt. Man erkennt, wie soeben beschrieben, am Anfang und am Ende der Darstellung Korrelationskoeffizientenwerte von ca. K = 1, und in der Mitte der Abbildung einen ausgeprägten "Einbruch" in der K-Kurve. Dieser Einbruch wird durch zwei charakteristische Parameter beschrieben, die sowohl unabhängig als auch in Kombination zur Identifizierung eines Treffers der Stoßwelle herangezogen werden können: Zum einen führt ein guter Treffer dazu, daß der Wert von K unter einen vorbestimmten Schwellenwert fällt, der in Fig. 5 durch eine horizontale gestrichelte Linie angedeutet ist. Andererseits konnte allgemein bei Stoßwellenbehandlungen festgestellt werden, daß die Relaxationszeit $T_R$, d.h. die Zeit, die vergeht, bis das System aus Konkrement 18 und herausgeschossenen Fragmenten wieder zur Ruhe gekommen ist, ein Maß für einen Treffer darstellt. Dementsprechend kann auch die Breite der K-Kurve entsprechend der Relaxationszeit $T_R$ als Maß für einen Treffer dienen. Daher ist die Auswerteeinheit 32 dazu ausgelegt, ein Fehlersignal zu erzeugen, wenn das Minimum des Korrelationskoeffizienten einen vorbestimmten ersten Schwellenwert nicht unterschreitet und/oder wenn die Relaxationszeit $T_R$ des Korrelationskoeffizienten einen vorbestimmten zweiten Schwellenwert unterschreitet. Es ist nämlich davon auszugehen, daß Fehl- bzw. Streifschüsse der Stoßwelle am Konkrement 18 vorbei allenfalls zu sehr schmalen Einbrüchen in der K-Kurve führen, wohingegen die Relaxationszeit $T_R$ um so größer sein wird, je präziser das Konkrement 18 von der Stoßwelle getroffen wurde.

Wie in Fig. 2 angedeutet, wird das von der Auswerteeinheit 32 erzeugte Fehlersignal dem Stoßwellengenerator 16 und einer Alarmvorrichtung 34 zugeführt. Der Stoßwellengenerator 16 kann somit im Fall von Fehlschüssen gegebenenfalls automatisch gestoppt werden. Auf diese Weise wird eine unnötige Belastung des Körpers des Patienten 12 durch die Stoßwellen-Fehlschüsse besonders sicher und wirksam vermieden, ohne daß es hierfür eines Eingreifens des medizinischen Personals bedürfte.

Die Alarmvorrichtung 34 kann einen akustischen und/oder optischen Alarm auslösen, wenn ein eingegebenes Fehlersignal auf einen Fehlschuß schließen läßt. In diesem Fall kann das medizinische Personal den Stoßwellengenerator 16 manuell abschalten oder andere Maßnahmen treffen, beispielsweise den Patienten 12 neu einjustieren.

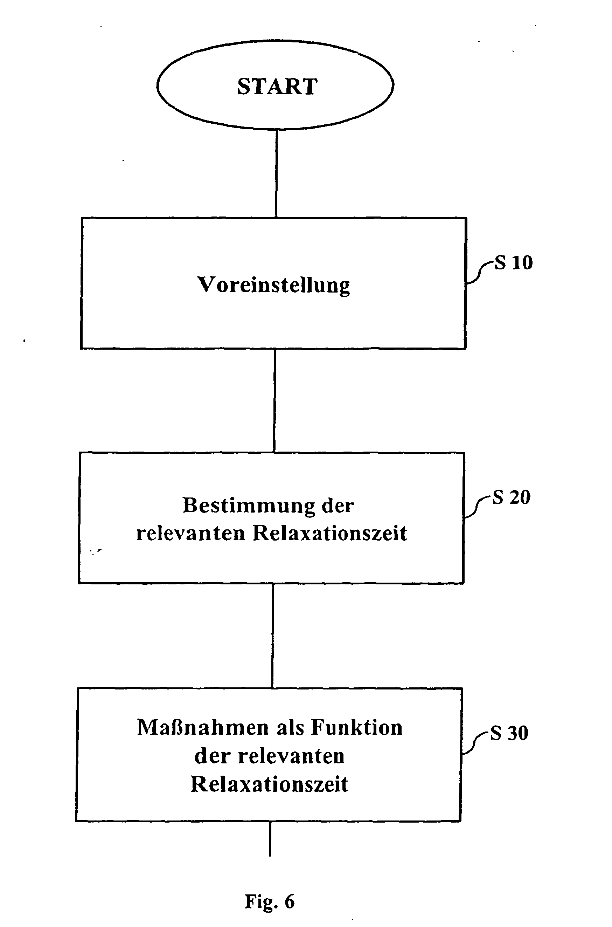

Wesentliche Schritte des mit dem erfindungsgemäßen Lithotripter 10 durchführbaren erfindungsgemäßen Verfahrens werden nachfolgend anhand der Fig. 6 bis 10 erläutert werden:

Wie in Fig. 6 schematisch angedeutet, umfaßt das erfindungsgemäße Verfahren nach dem Start des erfindungsgemäßen Lithotripters im wesentlichen drei Abschnitte, nämlich einen ersten Abschnitt S10 mit Schritten zur Voreinstellung des Lithotripters vor der eigentlichen Therapie, einen anschließenden Abschnitt S20, in dem während der Therapie in verschiedenen Verfahrensschritten die relevante Relaxationszeit $T_R$ bestimmt wird, sowie einen weiteren Abschnitt S30, in dem in mehreren Verfahrensschritten die entsprechenden Maßnahmen als Funktion der im Abschnitt S20 ermittelten relevanten Relaxationszeit $T_R$ ergriffen werden. Es sei bereits an dieser Stelle darauf hingewiesen, daß der Einfachheit halber im folgenden nur die erfaßte Relaxationszeit $T_R$ als Basis für die Trefferkontrolle herangezogen wird, selbstverständlich könnte jedoch auch nur das Minimum des Korrelationskoeffizienten K gemäß Fig. 5 oder auch eine Kombination beider Parameter von der Auswerteeinheit 32 herangezogen werden, um ein Fehlersignal zu erzeugen.

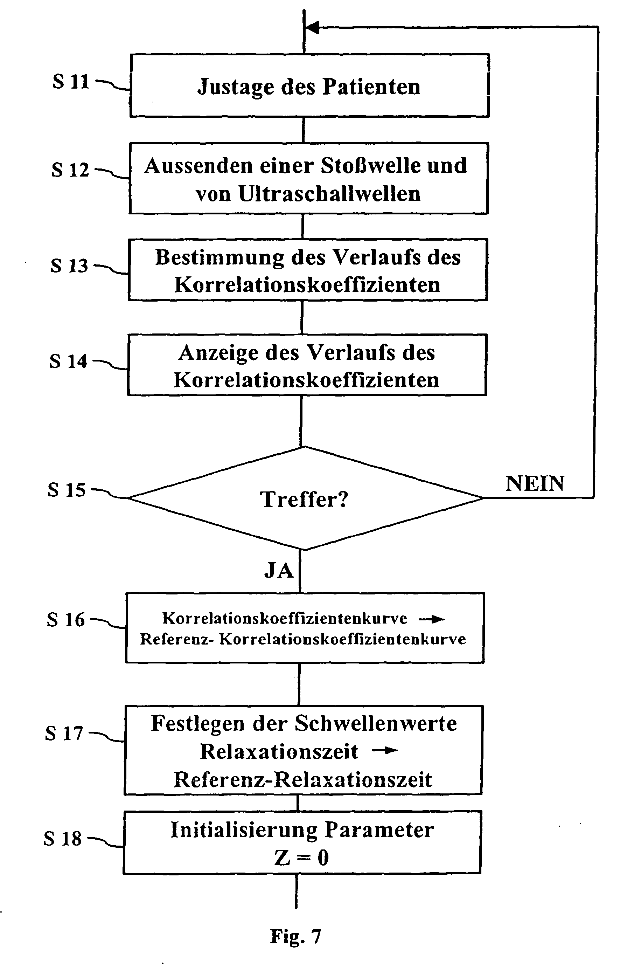

Die wichtigsten Schritte innerhalb des ersten Abschnitts S10 werden nachfolgend anhand von Fig. 7 erläutert werden:

Zunächst erfolgt im Schritt S11 die oben bereits genannte und im Stand der Technik bekannte Justage des Patienten mit Hilfe der verstellbaren Liege des Lithotripters 10. Am Ende dieser durch eine bildgebende Ortungsvorrichtung überwachten Justage ist der Patient 12 derart positioniert, daß sich sein Konkrement 18 im Stoßwellenfokus befindet.

Anschließend beginnt im Schritt S12 das Aussenden von Ultraschallwellen durch den Ultraschall-Transducer 24 sowie das erstmalige Aussenden einer Stoßwelle mit Hilfe des Stoßwellengenerators 16.

Anschließend bestimmt im Schritt S13 die Auswerteeinheit 32 anhand der reflektierten Ultraschallechos in der oben beschriebenen Weise den Verlauf des Korrelationskoeffizienten, welcher anschließend im Schritt S14 auf der Anzeigevorrichtung 30 entsprechend der Fig. 5 angezeigt wird. Bereits hierbei kann auf der Anzeigevorrichtung 30 auch eine Fit-Kurve angezeigt werden, die von der Auswerteeinheit 32 an den Einbruch in der K-Kurve angepaßt ("angefittet") wurde.

Dieser Verlauf des Korrelationskoeffizienten wird im Schritt S15 vom medizinischen Personal dahingehend begutachtet, ob er auf einen Treffer schließen läßt oder auf einen Fehlschuß. Bei diesem ersten Aussenden einer Stoßwelle im Schritt S12 ergibt sich in der Regel ein Treffer, da nach dem Schritt S11 das Konkrement 18 auf jeden Fall im Stoßwellenfokus liegt, und da man vor dem Aussenden dieser einer "Test-Stoßwelle" den Patienten 12 auffordern kann, kurzzeitig die Luft anzuhalten, so daß kein Fehlschuß aufgrund der Atmungsbewegung des Konkrements 18 droht. Bei dieser Begutachtung von Korrelationskoeffizientenverläufen dahingehend, ob ein Treffer oder ein Fehlschuß vorliegt, ist es unter anderem wichtig, das jeweilige Signalrauschen, d.h. Schwankungen zu ermitteln, die selbst bei Reflektion der Ultraschallwelle an einem ruhenden Patienten 12 ohne Aussenden von Stoßwellen auftreten.

Wird im Schritt S15 festgestellt, daß kein Treffer vorliegt, so kehrt man zu Schritt S11 zurück. Wird hingegen ein Treffer festgestellt, so wird im Schritt S16 die gemessene Korrelationskoeffizientenkurve als Referenz-Korrelationskoeffizientenkurve im Speicher 32a der Auswerteeinheit 32 abgespeichert. Mit Hilfe dieser Referenz-Korrelationskoeffizientenkurve werden dann im Schritt S17 die oben diskutierten Schwellenwerte festgelegt, deren Über- bzw. Unterschreiten dazu führt, daß die Auswerteeinheit 32 ein Fehlersignal ausgibt. Ferner wird im Schritt S17 die Relaxationszeit $T_R$, die bei der Referenz-Korrelationskoeffizientenkurve bestimmt wird, als Referenz-Relaxationszeit im Speicher 32a abgespeichert.

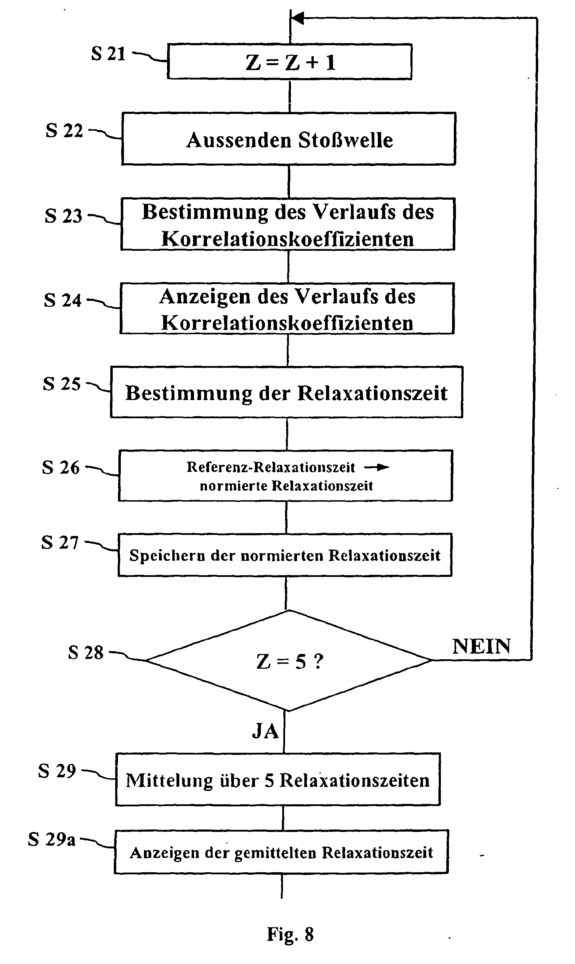

Anschließend erfolgt im Schritt S18 eine Initialisierung, d.h. ein Rücksetzen eines Zählparameters z, dessen Bedeutung nachfolgend anhand von Fig. 8 deutlich werden wird.

Fig. 8 stellt die wesentlichen Schritte innerhalb des zweiten Abschnitts S20 gemäß Fig. 6 dar, die zur Bestimmung der relevanten Relaxationszeit $T_R$ dienen.

Zunächst wird im Schritt S21 der Zählparameter z inkrementiert, d.h. um 1 erhöht. Anschließend wird im Schritt S22 eine Stoßwelle vom Stoßwellengenerator 16 ausgesandt und im Schritt S23 der Verlauf des Korrelationskoeffizienten bestimmt, der anschließend im Schritt S24 auf der Anzeigevorrichtung 30 angezeigt wird, ebenso wie eine gegebenenfalls angepaßte Fit-Kurve.

Im nachfolgenden Schritt S25 wird mit Hilfe der angefitteten Gauss-Kurve (selbstverständlich sind auch andere Fit-Kurven bzw. Auswertemethoden möglich, die auf dem Gebiet der Datenauswertung grundsätzlich bekannt sind), die Relaxationszeit $T_R$ dieses aktuellen Korrelationskoeffizientenverlaufs bestimmt. Im Schritt S26 wird diese Relaxationszeit $T_R$ mittels Division durch die im Schritt S17 gemäß Fig. 7 ermittelte Referenz-Relaxationszeit in eine normierte Relaxationszeit $T_R$ umgerechnet, die nachfolgend im Schritt S27 in der Auswerteeinheit 32 abgespeichert wird, beispielsweise in dem bereits genannten Speicher 32a oder in einem gesonderten Speicher zur Aufnahme von Meßwerten.

Dann wird im Schritt S28 überprüft, ob der Zählparameter z einen vorbestimmten Wert erreicht hat, bei dem in Fig. 8 gezeigten Beispiel den Wert z = 5. Ist dies nicht der Fall, so springt das in der Auswerteeinheit 32 ausgeführte Programm zum Schritt S21 zurück, in dem der Zählparameter z inkrementiert und anschließend ein weiterer Korrelationskoeffizientenverlauf gemessen wird. Ergibt die Prüfung im Schritt S28 jedoch, daß der Zählparameter z den vorbestimmten Wert, hier also z = 5 erreicht hat, was bedeutet, daß die Relaxationszeiten von 5 zurückliegenden Korrelationskoeffizientenverläufen in der Auswerteeinheit 32 zwischengespeichert sind, so geht das Programm weiter zu einem Schritt S29, in dem über diese fünf zwischengespeicherten Relaxationszeiten gemittelt wird. Der im Schritt S29 berechnete Mittelwert der Relaxationszeiten der letzten fünf Korrelationskoeffizientenverläufe kann ebenfalls in der Auswerteeinheit 32 in einem der genannten Speicher zwischengespeichert werden.

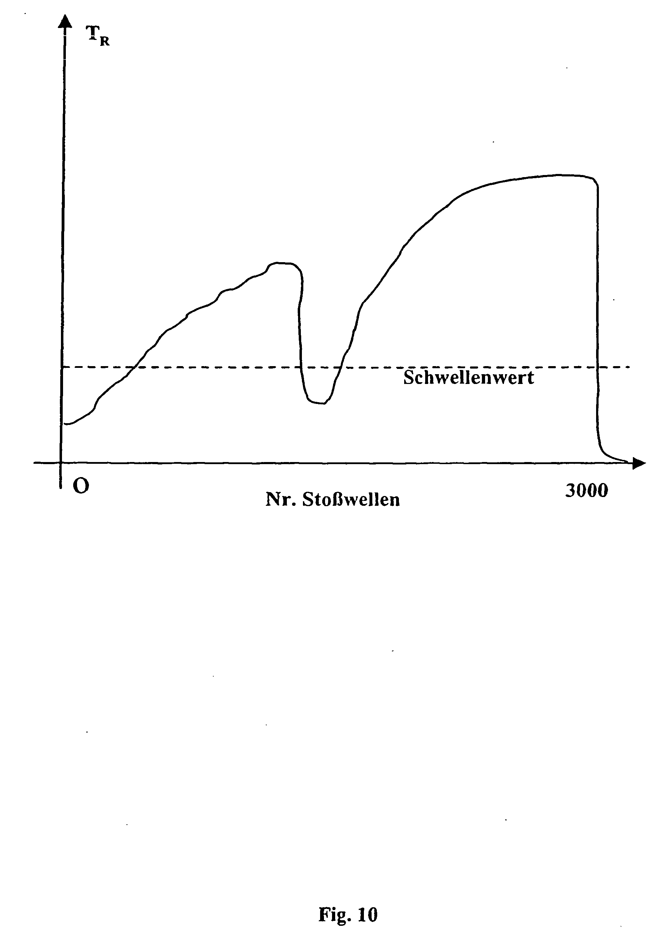

Im Schritt S29a wird der zuletzt berechnete Mittelwert einer fortlaufenden Anzeige hinzugefügt, welche die im Verlauf der gesamten Behandlung gemessenen Relaxationszeiten kontinuierlich darstellt, um die Entwicklung der Relaxationszeiten zu verfolgen. Zweckmäßigerweise kann diese fortlaufende Anzeige auf der Anzeigevorrichtung 30 dargestellt werden, es kann sich jedoch auch um einen gesonderten Bildschirm etc. handeln. Ein Beispiel für eine derartige fortlaufende Anzeige ist in Fig. 10 dargestellt und wird weiter unten erläutert werden.

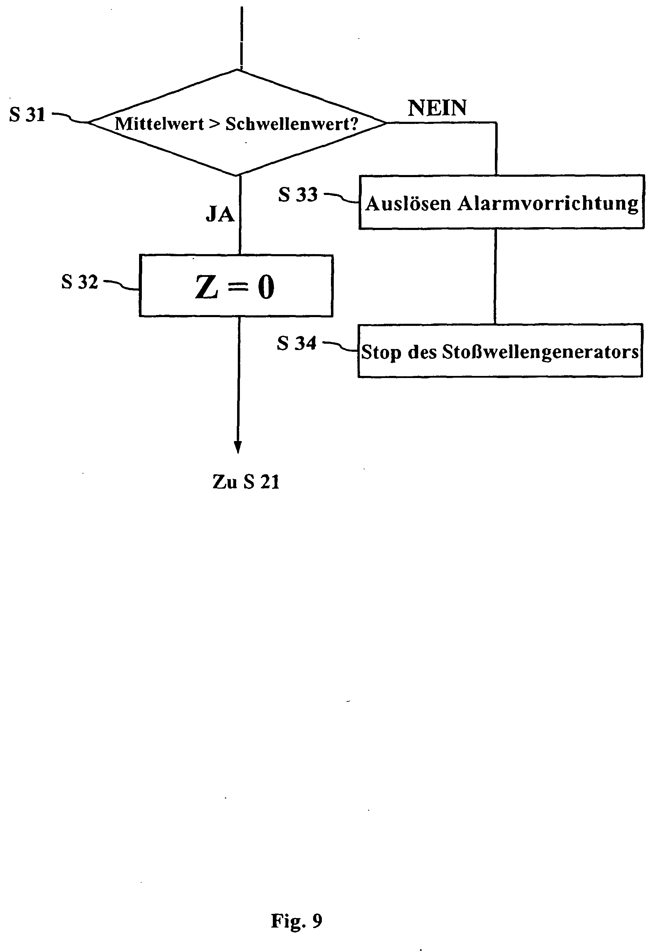

Fig. 9 zeigt die wesentlichen Schritte des dritten Abschnitts S30 gemäß Fig. 6 und die Maßnahmen, die im Rahmen des erfindungsgemäßen Verfahrens bei einer Lithotripsiebehandlung mit dem erfindungsgemäßen Lithotripter 10 als Funktion des im Schritt S29 berechneten Mittelwerts getroffen werden können:

In einem Schritt S31 wird durch das in der Auswerteeinheit 32 ausgeführte Programm überprüft, ob der im Schritt S29 berechnete Mittelwert größer als ein vorbestimmter Schwellenwert ist. Wie oben erläutert, deuten große Relaxationszeitenwerte auf Treffer hin, wohingegen kleine Relaxationszeitenwerte typisch für Fehlschüsse sind. Dementsprechend bedeutet ein positives Ergebnis der Prüfung im Schritt S31, das offensichtlich das Konkrement 18 weiterhin im Stoßwellenfokus liegt und somit Treffer erfolgen. Dementsprechend geht das in der Auswerteeinheit 32 ausgeführte Programm in diesem Fall über einen Schritt S32, in dem der Zählparameter z wiederum auf 0 zurückgesetzt wird, zurück zu Schritt S21, d.h. es beginnt erneut eine Messung von fünf normierten Relaxationszeiten mit anschließender Mittelung gemäß Fig. 8.

Führt die Prüfung im Schritt S31 jedoch zu einem negativen Ergebnis, d.h. dazu, daß die gemittelte Relaxationszeit $T_R$ der letzten fünf Korrelationskoeffizientenverläufe zu klein ist, was auf mögliche Fehlschüsse hinweist, so gibt die Auswerteeinheit 32 das besprochene Fehlersignal aus, und das Programm verzweigt zu einem Schritt S33, in dem die genannte Alarmvorrichtung 34 ausgelöst wird, sowie anschließend zu einem Schritt S34, in dem der Stoßwellengenerator 16 angehalten wird, um eine unnötige Belastung des Körpers des Patienten 12 durch Fehlschüsse zu vermeiden.

Fig. 10 ist eine schematische Darstellung einer beispielhaften Verlaufskontrollen-Anzeige auf der Anzeigevorrichtung 30 während einer Stoßwellentherapie. Man erkennt in Fig. 10 ein Koordinatensystem, dessen Abszisse die Zahl ausgesandter Stoßwellen und dessen Ordinate die im Anschluß an die jeweilige Stoßwelle ermittelte absolute oder normierte Relaxationszeit $T_R$ angibt. Man erkennt vom Start der Stoßwellentherapie bis kurz vor ihrem Ende einen Verlauf entsprechend einem langsamen Anstieg der Relaxationszeit $T_R$. Einzelne "Einbrüche" in dieser Verlaufskurve deuten auf Fehlschüsse durch eine Bewegung des Patienten 12 oder des Konkrements 18 innerhalb des Patienten 12 hin, die sofort durch eine Neupositionierung des Patienten 12 korrigiert wurden. In Fig. 9 repräsentiert eine horizontale gestrichelte Linie den Betrag des Schwellenwerts, dessen Unterschreiten die Alarmvorrichtung 34 auslöst. Man erkennt, daß der lokale Einbruch etwa in der Mitte der Verlaufskurve zu einem Unterschreiten dieses Schwellenwerts und somit zu einem Alarm geführt hat.

Es versteht sich, daß die Fig. 6 bis 10 nur die wichtigsten Schritte des erfindungsgemäßen Verfahrens angeben und zahlreiche vorherige, spätere oder auch eingeschobenen Schritte möglich sind, die auf dem Gebiet von Lithotripsieverfahren grundsätzlich bekannt sind. Ebenso versteht es sich, daß wie oben bereits erwähnt - anstelle der oder zusätzlich zur Relaxationszeit TR auch der Minimalwert des Korrelationskoeffizienten (vgl. Fig. 5) herangezogen werden kann, um einen Fehlschuß zu erkennen. Da in der Darstellung der Fig. 5 Treffer zu tiefen und gleichzeitig breiten Einbrüchen führen, kann auch die in dem "Einbruch" der K-Kurve enthaltenen Fläche numerisch bestimmt und als Maß für einen Treffer oder Fehlschuß herangezogen werden. Ferner versteht es sich nicht nur, daß die im Schritt S28 genannte Zahl z = 5 rein beispielhaft zu verstehen ist. Insbesondere kann diese Zahl auch im Rahmen der Voreinstellungen gemäß Abschnitt S10 in Fig. 6 vom medizinischen Person individuell eingegeben werden, beispielsweise im Schritt S17 in Fig. 7. Somit könnte beispielsweise bei einem Patienten 12 mit einem eher kleinen Konkrement 18 (das somit leichter durch die Stoßwelle zu verfehlen ist) und gleichzeitig einer schweren Atmung mit entsprechend starken Auslenkungen des Konkrements 18 über eine größere Zahl von Stoßwellen und somit Relaxationszeiten gemittelt werden als bei einem anderen Patienten 12 mit eher flacher Atmung und einem größeren Konkrement 18 (welches somit häufiger getroffen wird). Selbstverständlich kann auch ohne jede Mittelung gearbeitet werden, entsprechend z = 1.

Ebenso versteht es sich, daß die Ermittlung der Referenz-Relaxationszeit (Schritt S17) und die spätere Normierung gemessener Relaxationszeiten mit Hilfe dieser Referenz-Relaxationszeit (Schritt S26) auch unterbleiben kann, so daß bei der Verlaufskontrolle während der Stoßwellentherapie Absolutwerte der gemessenen Relaxationszeiten auf der Anzeigevorrichtung 20 angezeigt und entsprechend ausgewertet werden.

Hinsichtlich des anhand von Fig. 1 nur schematisch angedeuteten Lithotripters 10 versteht es sich, daß dieser zahlreiche weitere Komponenten aufweisen kann, die aus dem Stand der Technik bekannt sind, beispielsweise eine Röntgenortungsvorrichtung oder einen bildgebenden Ultraschallscanner.

## Bezugszeichenliste

| | |
|---|---|
| 10 | Lithotripter |
| 12 | Patient |
| 14 | Ankoppelkissten |
| 16 | Stoßwellengenerator |
| 18 | Konkrement (Nierenstein) |
| 20 | Niere |
| 22 | Stoßwellenquelle |
| 24 | Ultraschall-Transducer |
| 26 | Einstellbarer Halter |
| 28 | Steuereinheit |
| 30 | Anzeigevorrichtung |
| 32 | Auswerteeinheit |
| 32a | Speicher |
| 34 | Alarmvorrichtung |

## Patentansprüche

**1.** Lithotripter (10) zur Fragmentierung eines Zielobjekts (18), insbesondere eines Konkrements, in einem Körper, umfassend:

- einen Stoßwellengenerator (16) zur Erzeugung fokussierter Stoßwellen,
- eine Ultraschall-Sende-/Empfangseinheit mit einem Ultraschall-Transducer (24) zum Aussenden von Ultraschallwellen in den Körper und zum Empfangen von in einem Zielgebiet des Stoßwellengenerators (16) reflektierten Ultraschallwellen, wobei der Ultraschall-Transducer dazu ausgelegt ist, gepulste Ultraschallwellen auszusenden, und
- eine an die Ultraschall-Sende-/Empfangseinheit angeschlossene Auswerteeinheit (32) zum Auswerten der empfangenen Ultraschallwellen,

**dadurch gekennzeichnet, dass** die Auswerteeinheit (32) dazu ausgelegt ist, einen Korrelationskoeffizienten $K_{1,2}$ für eine zeitliche Korrelation zwischen einer ersten reflektierten Ultraschallwelle $e_1(t)$ und einer zweiten reflektierten Ultraschallwelle $e_2(t)$, die nacheinander ausgesandten Ultraschallpulsen zugeordnet sind, für ein bestimmtes Zeitintervall zu bestimmen und ein zugeordnetes Korrelationskoeffizientensignal auszugeben, wobei der Korrelationskoeffizient $K_{1,2}$ durch

$$\int_{T_1}^{T_2} e_1(t) * e_2(t)\,dt$$

und das Zeitintervall durch die Zeitpunkte $T_1$ und $T_2$ bestimmt wird.

2. Lithotripter (10) nach Anspruch 1, **dadurch gekennzeichnet, dass** die Auswerteeinheit (32) dazu ausgelegt ist, den Korrelationskoeffizienten $K_{1,2}$ anhand von reflektierten Ultraschallwellen zu bestimmen, die unmittelbar aufeinanderfolgenden ausgesandten Ultraschallpulsen zugeordnet sind.

3. Lithotripter (10) nach Anspruch 1 oder 2, **dadurch gekennzeichnet, dass** der Lithotripter ferner eine mit der Auswerteeinheit (32) verbundene Anzeigevorrichtung (30) zur Anzeige des zeitlichen Verlaufs des Korrelationskoeffizienten $K_{1,2}$ aufweist.

4. Lithotripter (10) nach einem der vorhergehenden Ansprüche, **dadurch gekennzeichnet, dass** die Auswerteeinheit (32) dazu ausgelegt ist, ein Fehlersignal auszugeben, wenn nach Aussenden einer Stoßwelle der Minimalwert des Korrelationskoeffizienten $K_{1,2}$ einen vorbestimmten ersten Schwellenwert nicht unterschreitet.

5. Lithotripter (10) nach einem der vorhergehenden Ansprüche, **dadurch gekennzeichnet, dass** die Auswerteeinheit (32) dazu ausgelegt ist, ein Fehlersignal auszugeben, wenn nach Aussenden einer Stoßwelle eine Relaxationszeit ($T_R$) des Korrelationskoeffizienten $K_{1,2}$ einen vorbestimmten zweiten Schwellenwert unterschreitet.

6. Lithotripter (10) nach Anspruch 5, **dadurch gekennzeichnet, dass** die Auswerteeinheit (32) dazu ausgelegt ist, die Relaxationszeit ($T_R$) durch Anpassen einer Fit-Kurve, z.B. einer Gauss-Kurve oder einer Kurve der Form $1 - A \exp(-t/T_R)$, an den zeitlichen Verlauf des Korrelationskoeffizienten $K_{1,2}$ zu bestimmen.

7. Lithotripter (10) nach einem der Ansprüche 4 bis 6, **dadurch gekennzeichnet, dass** er eine mit der Auswerteeinheit (32) verbundene Alarmvorrichtung (34) aufweist, der das Fehlersignal zugeführt wird.

8. Lithotripter (10) nach Anspruch 7, **dadurch gekennzeichnet, dass** die Alarmvorrichtung (34) zur Abgabe eines optischen und/oder akustischen Alarms ausgebildet ist.

9. Lithotripter (10) nach einem der Ansprüche 4 bis 8, **dadurch gekennzeichnet, dass** der Stoßwellengenerator (16) mit der Auswerteeinheit (32) verbunden und dazu ausgelegt ist, die Erzeugung von Stoßwellen als Funktion des Fehlersignals zu stoppen bzw. fortzusetzen.

10. Lithotripter (10) nach einem der Ansprüche 4 bis 9, **dadurch gekennzeichnet, dass** die Auswerteeinheit (32) Einstellmittel zum Einstellen des ersten und/oder des zweiten Schwellenwerts aufweist.

11. Lithotripter (10) nach einem der vorhergehenden Ansprüche, **dadurch gekennzeichnet, dass** die Auswerteeinheit (32) ferner dazu ausgelegt ist, den Verlauf der Werte des Korrelationskoeffizienten (K) zu glätten, insbesondere durch Mittelung.

12. Lithotripter (10) nach einem der vorhergehenden Ansprüche, **dadurch gekennzeichnet, dass** die Auswerteeinheit (32) ferner dazu ausgelegt ist, den Minimalwert und/oder die Relaxationszeit ($T_R$) des Korrelationskoeffizienten (K) über mehrere Stoßwellen zu mitteln.

13. Lithotripter (10) nach einem der vorhergehenden Ansprüche, **dadurch gekennzeichnet, dass** die Auswerteeinheit (32) ferner dazu ausgelegt ist, den Minimalwert und/oder die Relaxationszeit ($T_R$) des Korrelationskoeffizienten (K) auf den Referenz-Minimalwert bzw. die Referenz-Relaxationszeit einer Referenz-Korrelationskoeffizientenkurve zu normieren.

14. Lithotripter (10) nach einem der vorhergehenden Ansprüche, **dadurch gekennzeichnet, dass** die Auswerteeinheit (32) dazu ausgelegt ist, eine zeitliche Kreuzkorrelationsfunktion zwischen reflektierten Ultraschallwellen zu ermitteln und als Korrelationskoeffizienten den Maximalwert der zeitlichen Kreuzkorrelationsfunktion zu bestimmen.

15. Lithotripter (10) nach einem der vorhergehenden Ansprüche, **dadurch gekennzeichnet, dass** der Ultraschall-Transducer (24) der Ultraschall-Sende-/Empfangseinheit an einem einstellbaren Halter (26) montiert ist.

16. Lithotripter (10) nach einem der vorhergehenden Ansprüche, **dadurch gekennzeichnet, dass** die Ultraschall-Sende-/Empfangseinheit als Teil eines bildgebenden Ultraschallscanners ausgebildet ist.

17. Lithotripter (10) nach einem der vorhergehenden Ansprüche, **dadurch gekennzeichnet, dass** der Ultraschall-Transducer als Stiftsonde ausgebildet ist.

**18.** Lithotripter (10) nach Anspruch 17, **dadurch gekennzeichnet, dass** er ferner eine Röntgenortungsvorrichtung aufweist.

**19.** Lithotripter (10) nach einem der vorhergehenden Ansprüche, **dadurch gekennzeichnet, dass** er Mittel zur fortlaufenden Darstellung des Minimalwerts und/oder der Relaxationszeit ($T_R$) des Korrelationskoeffizienten über den Behandlungsverlauf umfasst.

**20.** Lithotripter (10) nach einem der vorhergehenden Ansprüche, **dadurch gekennzeichnet, dass** der Körper ein menschlicher Körper ist.

**21.** Lithotripter (10) nach einem der vorhergehenden Ansprüche, **dadurch gekennzeichnet, dass** der Korrelationskoeffizient $K_{1,2}$ mit dem Faktor $\left(\int_{T_1}^{T_2} e_1^2(t)dt\right)^{1/2}\left(\int_{T_1}^{T_2} e_2^2(t)dt\right)^{1/2}$ normiert wird.

**22.** Verfahren zur Überwachung der Fragmentierung eines Zielobjekts (18), insbesondere eines Konkrements, in einem Körper, umfassend die Schritte:

- Aussenden von gepulste Ultraschallwellen in den Körper, und,
- Empfangen von am Zielobjekt (18) reflektierten Ultraschallwellen, und
- Auswerten der empfangenen Ultraschallwellen,

**dadurch gekennzeichnet, dass** der Schritt des Auswertens eine Bestimmung eines Korrelationskoeffizienten $K_{1,2}$ für eine zeitliche Korrelation zwischen einer ersten reflektierten Ultraschallwelle $e_1(t)$ und einer zweiten reflektierten Ultraschallwelle $e_2(t)$, die nacheinander ausgesandten Ultraschallpulsen zugeordnet sind, für ein bestimmtes Zeitintervall umfasst und dass ferner ein Schritt der Ausgabe eines zugeordneten Korrelationskoeffizientensignals vorgesehen ist, wobei der Korrelationskoeffizient $K_{1,2}$ durch $\int_{T_1}^{T_2} e_1(t)*e_2(t)dt$ und das Zeitintervall durch die Zeitpunkte $T_1$ und $T_2$ bestimmt wird.

**23.** Verfahren nach Anspruch 22, **gekennzeichnet durch** den weiteren Schritt des fortlaufenden Darstellens des Minimalwerts und/oder der Relaxationszeit ($T_R$) des Korrelationskoeffizienten $K_{1,2}$ über den Behandlungsverlauf.

**24.** Verfahren nach einem der vorhergehenden Ansprüche, **dadurch gekennzeichnet, dass** der Korrelationskoeffizient $K_{1,2}$ mit dem Faktor $\left(\int_{T_1}^{T_2} e_1^2(t)dt\right)^{1/2}\left(\int_{T_1}^{T_2} e_2^2(t)dt\right)^{1/2}$ normiert wird..

**Claims**

**1.** A lithotripter (10) for fragmenting a target object (18), particularly a concrement, in a body, comprising:

- a shockwave generator (16) for generating focused shockwaves,
- an ultrasonic transmitting/receiving unit including an ultrasonic transducer (24) for emitting ultrasonic waves into the body and for receiving ultrasonic waves reflected in a target area of said shockwave generator (16), said ultrasonic transducer (24) being designed to emit pulsed ultrasonic waves, and
- an evaluating unit (32) connected to said ultrasonic transmitting/receiving unit for evaluating the received ultrasonic waves,

**characterized in that** said evaluating unit (32) is designed to determine a correlation coefficient $K_{1,2}$ for a time correlation between a first reflected ultrasonic wave $e_1(t)$ and a second reflected ultrasonic wave $e_2(t)$ assigned to

successively emitted ultrasonic pulses, for a specific time interval, and to provide an associated correlation coefficient signal, the correlation coefficient K1,2 being determined by

$$\int_{T_1}^{T_2} e_1(t) * e_2(t) dt$$

and the time interval by the times $T_1$ and $T_2$.

2. The lithotripter (10) according to claim 1, **characterized in that** said evaluating unit (32) is designed to determine the correlation coefficient $K_{1,2}$ on the basis of reflected ultrasonic waves assigned to emitted ultrasonic pulses directly succeeding one another.

3. The lithotripter (10) according to claim 1 or 2, **characterized in that** it further comprises a display device (30) connected to said evaluating unit (32) for displaying the variation of the correlation coefficient $K_{1,2}$ with time.

4. The lithotripter (10) according to any one of the preceding claims, **characterized in that** said evaluating unit (32) is designed to provide an error signal if after emission of a shockwave the minimum value of said correlation coefficient $K_{1,2}$ does not fall below a predetermined first threshold value.

5. The lithotripter (10) according to any one of the preceding claims, **characterized in that** said evaluating unit (32) is designed to provide an error signal if after emission of a shockwave a relaxation time ($T_R$) of said correlation coefficient $K_{1,2}$ falls below a predetermined second threshold value.

6. The lithotripter (10) according to claim 5, **characterized in that** said evaluating unit (32) is designed to determine the relaxation time ($T_R$) by adapting a fit curve, e.g. a Gauss curve, or a curve of the form 1 - A exp(-t/$T_R$), to the variation of said correlation coefficient $K_{1,2}$ with time.

7. The lithotripter (10) according to any one of claims 4 to 6, **characterized in that** it comprises an alarm device (34) which is connected to said evaluating unit (32) and is supplied with said error signal.

8. The lithotripter (10) according to claim 7, **characterized in that** said alarm device (34) is designed for outputting an optical and/or acoustic alarm.

9. The lithotripter (10) according to any one of claims 4 to 8, **characterized in that** said shockwave generator (16) is connected to said evaluating unit (32) and designed to stop or continue the generation of shockwaves as a function of the error signal.

10. The lithotripter (10) according to any one of claims 4 to 9, **characterized in that** said evaluating unit (32) comprises adjusting means for adjusting the first and/or second threshold value.

11. The lithotripter (10) according to any one of the preceding claims, **characterized in that** said evaluating unit (32) is further designed to smooth the variation of the values of said correlation coefficient (K), particularly by way of averaging.

12. The lithotripter (10) according to any one of the preceding claims, **characterized in that** said evaluating unit (32) is further designed to average the minimum value and/or the relaxation time ($T_R$) of said correlation coefficient (K) over a plurality of shockwaves.

13. The lithotripter (10) according to any one of the preceding claims, **characterized in that** said evaluating unit (32) is further designed to normalize the minimum value and/or the relaxation time ($T_R$) of said correlation coefficient (K) to the reference minimum value or the reference relaxation time of a reference correlation coefficient curve.

14. The lithotripter (10) according to any one of the preceding claims, **characterized in that** said evaluating unit (32) is designed to determine a temporal cross correlation function between reflected ultrasonic waves and to define the maximum value of said temporal cross correlation function as said correlation coefficient.

15. The lithotripter (10) according to any one of the preceding claims, **characterized in that** said ultrasonic transducer (24) of said ultrasonic transmitting/receiving unit is mounted on an adjustable holder (26).

16. The lithotripter (10) according to any one of the preceding claims, **characterized in that** said ultrasonic transmitting/ receiving unit is designed as part of an imaging ultrasonic scanner.

17. The lithotripter (10) according to any one of the preceding claims, **characterized in that** said ultrasonic transducer is designed as a pin probe.

18. The lithotripter (10) according to claim 17, **characterized in that** it further comprises an X-ray locating device.

19. The lithotripter (10) according to any one of the preceding claims, **characterized in that** it comprises means for the continuous representation of the minimum value and/or the relaxation time ($T_R$) of said correlation coefficient over the treatment duration.

20. The lithotripter (10) according to any one of the preceding claims, **characterized in that** the body is a human body.

21. The lithotripter (10) according to any one of the preceding claims, **characterized in that** the correlation coefficient $K_{1,2}$ is normalized with the factor

$$\left( \int_{T_1}^{T_2} e_1^2(t)dt \right)^{1/2} \left( \int_{T_1}^{T_2} e_2^2(t)dt \right)^{1/2}$$

22. A method for monitoring the fragmentation of a target object (18), particularly a concrement, in a body, comprising the steps of:

- emitting pulsed ultrasonic waves into said body, and
- receiving ultrasonic waves reflected on said target object (18), and
- evaluating the received ultrasonic waves,

**characterized in that** the evaluating step comprises a determination of a correlation coefficient $K_{1,2}$ for a time correlation between a first reflected ultrasonic wave $e_1(t)$ and a second reflected ultrasonic wave $e_2(t)$ assigned to successively emitted ultrasonic pulses, for a specific time interval, and that further a step of outputting an associated correlation coefficient signal is provided, the correlation coefficient $K_{1,2}$ being determined by

$$\int_{T_1}^{T_2} e_1(t) * e_2(t)dt$$

and the time interval by the times $T_1$ and $T_2$.

23. The method according to claim 22, **characterized by** the further step of continuously representing the minimum value and/or the relaxation time($T_R$) of said correlation coefficient $K_{1,2}$ over the treatment duration.

24. The method according to any one of the preceding claims, **characterized in that** the correlation coefficient $K_{1,2}$ is normalized with the factor

$$\left( \int_{T_1}^{T_2} e_1^2(t)dt \right)^{1/2} \left( \int_{T_1}^{T_2} e_2^2(t)dt \right)^{1/2} .$$

**Revendications**

1. Lithotripteur (10) pour la fragmentation d'un objet-cible (18), en particulier d'une concrétion dans un corps, comprenant :

   - un générateur d'ondes de choc (16) pour la génération d'ondes de choc focalisées,
   - une unité d'émission-réception d'ultrasons, avec un transducteur à ultrasons (24) pour l'émission d'ondes ultrasoniques vers le corps et pour la réception d'ondes ultrasoniques réfléchies dans une zone-cible du générateur d'ondes de choc (16), le transducteur à ultrasons étant prévu pour émettre des ondes ultrasoniques à impulsions, et
   - une unité d'analyse (32) reliée à l'unité d'émission/réception pour l'analyse des ondes ultrasoniques reçues,

   **caractérisé en ce que** l'unité d'analyse (32) est prévue pour déterminer un coefficient de corrélation $K_{1,2}$ pour une corrélation temporelle entre une première onde ultrasonique réfléchie $e_1(t)$ et une deuxième onde ultrasonique réfléchie $e_2(t)$, associées à des impulsions ultrasoniques émises l'une après l'autre, pendant un intervalle temporel défini et pour émettre un signal de coefficient de corrélation correspondant, le coefficient de corrélation $K_{1,2}$ étant déterminé par $\int_{T_1}^{T_2} e_1(t) * e_2(t) dt$ et l'intervalle temporel par les moments $T_1$ et $T_2$.

2. Lithotripteur (10) selon la revendication 1, **caractérisé en ce que** l'unité d'analyse (32) est prévue pour déterminer le coefficient de corrélation $K_{1,2}$ à partir d'ondes ultrasoniques réfléchies associées à des impulsions ultrasoniques émises l'une immédiatement après l'autre.

3. Lithotripteur (10) selon la revendication 1 ou 2, **caractérisé en ce que** le lithotripteur comporte en outre un dispositif afficheur (30) relié à l'unité d'analyse (32), pour l'affichage de la courbe temporelle du coefficient de corrélation $K_{1,2}$.

4. Lithotripteur (10) selon l'une des revendications précédentes, **caractérisé en ce que** l'unité d'analyse (32) est prévue pour émettre un signal d'erreur si, après émission d'une onde de choc, la valeur minimale du coefficient de corrélation $K_{1,2}$ ne devient pas inférieure à une première valeur de seuil prédéfinie.

5. Lithotripteur (10) selon l'une des revendications précédentes, **caractérisé en ce que** l'unité d'analyse (32) est prévue pour émettre un signal d'erreur si, après émission d'une onde de choc, un temps de relaxation ($T_R$) du coefficient de corrélation $K_{1,2}$ devient inférieur à une deuxième valeur seuil prédéfinie.

6. Lithotripteur (10) selon la revendication 5, **caractérisé en ce que** l'unité d'analyse (32) est prévue pour déterminer le temps de relaxation ($T_R$) par adaptation d'une courbe d'ajustement telle qu'une courbe de Gauss ou une courbe de forme 1 - A exp(-t/$T_R$) à la courbe temporelle du coefficient de corrélation $K_{1,2}$.

7. Lithotripteur (10) selon l'une des revendications 4 à 6, **caractérisé en ce qu'**il comporte un dispositif d'alerte (34) relié à l'unité d'analyse (32), auquel le signal d'erreur est transmis.

8. Lithotripteur (10) selon la revendication 7, **caractérisé en ce que** le dispositif d'alerte (34) est prévu pour l'émission d'une alerte optique et/ou acoustique.

9. Lithotripteur (10) selon l'une des revendications 4 à 8, **caractérisé en ce que** le générateur d'ondes de choc (16) est relié à l'unité d'analyse (32) et est prévu pour arrêter ou poursuivre la génération d'ondes de choc en fonction du signal d'erreur.

10. Lithotripteur (10) selon l'une des revendications 4 à 9, **caractérisé en ce que** l'unité d'analyse (32) comporte des moyens de réglage pour le réglage de la première et/ou de la deuxième valeur de seuil.

11. Lithotripteur (10) selon l'une des revendications précédentes, **caractérisé en ce que** l'unité d'analyse (32) est en outre prévue pour lisser la courbe de valeurs du coefficient de corrélation (K), en particulier par formation de moyenne.

**12.** Lithotripteur (10) selon l'une des revendications précédentes, **caractérisé en ce que** l'unité d'évaluation (32) est en outre prévue pour former la moyenne de la valeur minimale et/ou du temps de relaxation ($T_R$) du coefficient de corrélation (K) sur plusieurs ondes de choc.

**13.** Lithotripteur (10) selon l'une des revendications précédentes, **caractérisé en ce que** l'unité d'évaluation (32) est en outre prévue pour normaliser la valeur minimale et/ou le temps de relaxation ($T_R$) du coefficient de corrélation (K) à la valeur minimale de référence ou le temps de relaxation de référence d'une courbe de coefficient de corrélation de référence.

**14.** Lithotripteur (10) selon l'une des revendications précédentes, **caractérisé en ce que** l'unité d'évaluation (32) est prévue pour saisir une fonction de corrélation temporelle croisée entre des ondes ultrasoniques réfléchies et à définir comme coefficient de corrélation la valeur maximale de la fonction de corrélation temporelle croisée.

**15.** Lithotripteur (10) selon l'une des revendications précédentes, **caractérisé en ce que** le transducteur à ultrasons (24) de l'unité d'émission/réception d'ultrasons est monté sur un support réglable (26).

**16.** Lithotripteur (10) selon l'une des revendications précédentes, **caractérisé en ce que** l'unité d'émission/réception d'ultrasons est prévue comme partie d'un scanner à ultrasons restituteur d'image.

**17.** Lithotripteur (10) selon l'une des revendications précédentes, **caractérisé en ce que** le transducteur à ultrasons est réalisé comme sonde à tige.

**18.** Lithotripteur (10) selon la revendication 17, **caractérisé en ce qu'**il comporte en outre un dispositif de localisation radiographique.

**19.** Lithotripteur (10) selon l'une des revendications précédentes, **caractérisé en ce qu'**il comprend des moyens pour la représentation continue de la valeur minimale et/ou du temps de relaxation ($T_R$) du coefficient de corrélation (K) pendant la durée du traitement.

**20.** Lithotripteur (10) selon l'une des revendications précédentes, **caractérisé en ce que** le corps est un corps humain.

**21.** Lithotripteur (10) selon l'une des revendications précédentes, **caractérisé en ce que** le coefficient de corrélation

$K_{1,2}$ est normalisé par le facteur $\left( \int_{T_1}^{T_2} e_1^2(t)dt \right)^{1/2} \left( \int_{T_1}^{T_2} e_2^2(t)dt \right)^{1/2}$ .

**22.** Procédé de surveillance de la fragmentation d'un objet-cible (18), en particulier d'une concrétion dans un corps, comprenant les étapes suivantes :

- émission d'ondes ultrasoniques à impulsions vers le corps, et
- réception d'ondes ultrasoniques réfléchies sur l'objet-cible (18), et
- analyse des ondes ultrasoniques reçues,

**caractérisé en ce que** l'étape d'analyse comprend une détermination d'un coefficient de corrélation $K_{1,2}$ pour une corrélation temporelle entre une première onde ultrasonique réfléchie $e_1(t)$ et une deuxième onde ultrasonique réfléchie $e_2(t)$, associées à des impulsions ultrasoniques émises l'une après l'autre, pendant un intervalle temporel défini, et **en ce qu'**une étape d'émission d'un signal de coefficient de corrélation correspondant est en outre prévue, le coefficient de corrélation $K_{1,2}$ étant déterminé par $\int_{T_1}^{T_2} e_1(t) * e_2(t)dt$ et l'intervalle temporel par les moments $T_1$ et $T_2$.

**23.** Procédé selon la revendication 22, **caractérisé par** étape supplémentaire de représentation continue de la valeur minimale et/ou du temps de relaxation ($T_R$) du coefficient de corrélation $K_{1,2}$ pendant la durée du traitement.

**24.** Procédé selon l'une des revendications précédentes, **caractérisé en ce que** le coefficient de corrélation $K_{1,2}$ est normalisé par le facteur $\left( \int_{T_1}^{T_2} e_1^2(t)dt \right)^{1/2} \left( \int_{T_1}^{T_2} e_2^2(t)dt \right)^{1/2}$ .

Fig. 1

**Fig. 2**

Fig. 3

**Fig. 4**

**Fig. 5**

START

Voreinstellung — S 10

Bestimmung der
relevanten Relaxationszeit — S 20

Maßnahmen als Funktion
der relevanten
Relaxationszeit — S 30

Fig. 6

S 11 — | Justage des Patienten |

S 12 — | Aussenden einer Stoßwelle und von Ultraschallwellen |

S 13 — | Bestimmung des Verlaufs des Korrelationskoeffizienten |

S 14 — | Anzeige des Verlaufs des Korrelationskoeffizienten |

S 15 — Treffer?  NEIN

JA

S 16 — | Korrelationskoeffizientenkurve →
Referenz- Korrelationskoeffizientenkurve |

S 17 — | Festlegen der Schwellenwerte
Relaxationszeit →
Referenz-Relaxationszeit |

S 18 — | Initialisierung Parameter
Z = 0 |

Fig. 7

S 21 — $Z = Z + 1$

S 22 — Aussenden Stoßwelle

S 23 — Bestimmung des Verlaufs des Korrelationskoeffizienten

S 24 — Anzeigen des Verlaufs des Korrelationskoeffizienten

S 25 — Bestimmung der Relaxationszeit

S 26 — Referenz-Relaxationszeit → normierte Relaxationszeit

S 27 — Speichern der normierten Relaxationszeit

S 28 — $Z = 5$ ?  NEIN

JA

S 29 — Mittelung über 5 Relaxationszeiten

S 29a — Anzeigen der gemittelten Relaxationszeit

Fig. 8

S 31 — Mittelwert > Schwellenwert? ———— NEIN

JA

S 33 — Auslösen Alarmvorrichtung

S 32 — Z = 0

S 34 — Stop des Stoßwellengenerators

Zu S 21

**Fig. 9**

Fig. 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0367116 B1
- EP 0548048 B1
- DE 4446192 A1

- US 5287856 A
- US 4819621 A

**In der Beschreibung aufgeführte Nicht-Patentliteratur**

- Flow Velocity Profile via Time-Domain Correlation: Error Analysis and Computer Simulation. **VON STEVEN G. FOSTER ; PAUL M. EMBREE ; WILLIAM D. O'BRIEN JR.** IEEE Transactions on Ultrasonics. Ferroelectrics and Frequency Control, Mai 1990, vol. 37

- **VON O. BONNEFOUS ; P. PESQUE.** Time Domain Formulation of Pulse-Doppler Ultrasound and Blood Velocity Estimation by Cross Correlation. *Ultrasonic Imaging,* 1986, vol. 8, 73-85